# EUROPÄISCHE PATENTSCHRIFT

(11) **EP 4 267 935 B1**
(45) Veröffentlichungstag und Bekanntmachung des Hinweises auf die Patenterteilung: **03.12.2025**
(21) Anmeldenummer: 21844023.8
(22) Anmeldetag: 23.12.2021
(51) Int. Cl.: G01N 1/40, G01N 33/49

(54) **VORRICHTUNG UND VERFAHREN ZUR SEPARATION VON UNTERSCHIEDLICH GROSSEN PARTIKELN IN EINER FLÜSSIGKEIT UND VERWENDUNGEN DER VORRICHTUNG**
DEVICE AND METHOD FOR SEPARATING PARTICLES OF DIFFERENT SIZES IN A LIQUID, AND APPLICATIONS OF THE DEVICE
DISPOSITIF ET PROCÉDÉ DE SÉPARATION DE PARTICULES DE DIFFÉRENTES TAILLES DANS UN LIQUIDE, ET APPLICATIONS DU DISPOSITIF

(30) Priorität: 28.12.2020 DE 102020216578
(43) Veröffentlichungstag der Anmeldung: 01.11.2023
(73) Patentinhaber: Fraunhofer-Gesellschaft zur Förderung der angewandten Forschung e.V., 80686 München (DE)
(72) Erfinder: SCHMIEDER, Florian, 01277 Dresden (DE)
(74) Vertreter: Pfenning, Meinig & Partner mbB
(86) Internationale Anmeldenummer: PCT/EP2021/087577
(87) Internationale Veröffentlichungsnummer: WO 2022/144314

(56) Entgegenhaltungen:
- US-A- 3 972 812
- US-A- 6 139 757
- US-B1- 6 312 950

## Beschreibung

Es wird eine Vorrichtung und ein Verfahren zur Separation von unterschiedlich großen Partikeln in einer Flüssigkeit bereitgestellt. Ferner werden Verwendungen der erfindungsgemäßen Vorrichtung vorgeschlagen. Die erfindungsgemäße Vorrichtung und das erfindungsgemäße Verfahren beruhen darauf, dass der Porendurchmesser der Poren des mindestens einen Filterelements der Vorrichtung gezielt verändert (z.B. vergrößert oder verkleinert) werden kann. Die Vorrichtung und das Verfahren haben den Vorteil, dass unterschiedlich große Partikel (z.B. biologische Zellen und/oder Endosomen) einer Flüssigkeit mit einer hohen Trennschärfe voneinander getrennt werden können und die Separation der Partikel auf eine einfache, schnelle und kostengünstige Art und Weise erfolgt, wobei hohen Ausbeuten realisiert werden können und die separierten Partikel in einer therapeutisch verwendbaren Flüssigkeit (z.B. Blutplasma) bereitgestellt werden können.

Die Separation von unterschiedlich großen Partikeln in einer Flüssigkeit (z.B. einer Biosuspension enthaltend biologische Zellen und/oder Krankheitserreger) ist für viele medizintechnische Fragestellungen (z.B. die Gewinnung von Blutzellpräparaten, die labortechnische Analyse einzelner Komponenten und/oder die Aufbereitung von Zellpräparaten für zelltherapeutische Maßnahmen) von großem Interesse.

Zwei Grundprinzipien zur Separation haben sich dabei besonders etabliert. Das erste Grundprinzip trennt Partikel, die in einer Flüssigkeit vorliegen, anhand ihrer unterschiedlichen spezifischen Dichte oder anhand ihrer unterschiedlichen mechanischen Eigenschaften (z.B. ihre Deformierbarkeit und/oder Ausrichtung in einem Flüssigkeitsstrom). Dazu wird generell eine Filtration und/oder Zentrifugation verwendet. Das zweite Grundprinzip trennt Partikel anhand ihrer unterschiedlichen Oberflächeneigenschaften. Im Falle von Zellen, Viren und/oder Endosomen als Partikel werden diese unterschiedlichen Oberflächeneigenschaften durch eine Exponierung unterschiedlicher Moleküle (z.B. Proteine, Lipide und/oder Zucker) an der Oberfläche dieser Partikel hervorgerufen.

Im Stand der Technik sind bereits Vorrichtungen und Verfahren bekannt, um unterschiedlich große Partikel in einer Flüssigkeit voneinander zu separieren.

Erstens ist die Zentrifugation bekannt. Beispielsweise wird diese zur Aufreinigung von Vollblut verwendet, im Wesentlichen zur Herstellung von Blutpräparaten für den therapeutischen Einsatz. Dabei werden verschiedene Separationsarten unterschieden, wobei diese immer aus einer Abfolge von mehreren Zentrifugationsschritten bestehen. Das Vollblut wird üblicherweise direkt im Blutentnahmebeutel zentrifugiert und in einer separaten Maschine auf weitere Beutel verteilt, die mit diesem Beutel über Schläuche verbunden sind. Die Endprodukte sind Erythrozyten (sog. "packed RBC") plättchenreiches oder plättchenarmes Blutplasma, Blutplättchen sowie periphere monokulare Zellen (sog. "PBMC"). Der größte Nachteil dieses etablierten Verfahrens liegt in der geringen Reinheit der erzielten Endprodukte, insbesondere der PBMC, die im sog. "buffy coat" vorliegen.

Zweitens ist die Dichtegradientenzentrifugation bekannt. Beispielsweise kommt die Dichtegradientenzentrifugation von Vollblut im Wesentlichen im diagnostischen Bereich zum Einsatz. Dies liegt daran, dass das bei diesem Verfahren verwendete Separationsmedium am Ende des Verfahrens nicht vollständig abgetrennt werden kann, was die bereitgestellten, separierten Zellsuspensionen für den therapeutischen Einsatz ungeeignet macht. Generell läuft das Verfahren ähnlich der Zentrifugation ab. Vor diesem Verfahren wird das Blut jedoch mit einem Separationsmedium unterschichtet, dessen Dichte zwischen der Dichte der PBMC und der der Erythrozyten sowie Granulozyten liegt. Damit entsteht in der Separation eine Trennphase zwischen einer Erythrozyten/Granulozyten-Phase und einer PBMC-Phase, die eine bessere Separation der PBMC ermöglicht. Das gewünschte Endprodukt der Separation sind meist die PBMC. Die Nachteile des Verfahrens liegen in der mangelnden Reinheit der Endprodukte, der mangelnden Ausbeute, sowie der komplizierten Handhabung beim Abpipettieren der Zellen.

Drittens ist eine mikrofluidische Separation über Microarrays bekannt. In diesen Verfahren wird eine Separation von Blutzellen über einen Fluss der Blutzellsuspension durch ein Mikrogitter erreicht, das in eine mikrofluidische Kartusche integriert ist. Mit solchen Kartuschen können hohe Reinheiten bezüglich der Separation von PBMC erzielt werden. Nachteilig an diesem Verfahren ist jedoch die komplizierten Ansteuerung sowie eine sehr lange Verfahrensdauer (für 400 mL Vollblut beträgt die Dauer der Separation mit Mikrofluidik ca. 3 Stunden).

Viertens ist die Plasmaapherese bekannt, die eine Zentrifugation in einem kontinuierlichen Verfahren darstellt. Dabei wird Blut während der Blutentnahme kontinuierlich mit Antikoagulantien versetzt und durch eine Pumpe in ein rotierendes Zentrifugationsgefäß gefördert. Die vom Plasma getrennten Zellen werden aufgefangen, mit physiologischer Salzlösung isotonisch ausgeglichen und anschließend über eine weitere Pumpe dem Patienten wieder zugeführt. Der größte Nachteil des Verfahrens ist, dass nur zwei Fraktionen des Bluts kontinuierlich getrennt werden können und somit keine feine Aufteilung in mehr als zwei Zellfraktionen erfolgen kann.

Fünftens ist die Filtration bekannt. In diesen Verfahren wird gewöhnlich entweder eine Filtermembran zur Plasmaseparation oder eine Filtermembran zur Separation von Zellfraktionen wie beispielsweise PBMC. Idealerweise ist die verwendete Filtermembran direkt in einem Gefäß verbaut, um Flüssigkeit mit den Partikeln, welche die Membran passieren, aufzufangen. Außerdem sind Separationsgefäße bekannt, in denen eine Kaskade von Filtern verwendet wird, um unterschiedliche Fraktionen voneinander zu isolieren. Weiterhin ist ein Verfahren bekannt, in dem Zellen durch ein Einbringen von porösen Mikrowürfeln in die Suspension in diesen Mikrowürfeln eingeschlossen werden. Die derzeit bekannten Filtrationsverfahren haben den Nachteil, dass nur eine sehr grobe Separation von Partikeln einer Flüssigkeit möglich ist, d.h. letztlich nur zwei Arten von Partikeln voneinander getrennt werden können, nämlich Partikel, deren Durchmesser kleiner ist als ein Porendurchmesser der Poren der Filtermembran von Partikeln, deren Durchmesser größer ist als ein Porendurchmesser der Poren der Filtermembran. Um eine höhere Trennschärfe zu erreichen, müssen weitere Maßnahmen getroffen werden (z.B. Absorption von bestimmten Partikeln in Mikrowürfeln), die zeitintensiv und kostenintensiv sind und die Ausbeute an separierten Zellen verringern.

Die US 6 312 950 B1 offenbart eine Vorrichtung und Verfahren zum Isolieren und Rückgewinnen einer Zielzelle. Die Vorrichtung enthält eine Filtervorrichtung mit einem Filterelement, dessen Porosität auf zwei oder mehr verschiedene Porositäten eingestellt werden kann.

Die US 6 139 757 A offenbart ein Verfahren zur Abtrennung von Zellen aus Blut unter Verwendung einer Filtervorrichtung mit einer ersten und einer zweiten Öffnung und einem dazwischenliegenden Filterelement mit veränderlicher Porosität, wobei das Filterelement einen Stapel poröser Polymerschichten umfasst.

Ausgehend hiervon war es die Aufgabe der vorliegenden Erfindung, eine Vorrichtung und ein Verfahren zur Separation von unterschiedlich großen Partikeln in einer Flüssigkeit bereitzustellen, welche die Nachteile aus dem Stand der Technik nicht aufweisen. Insbesondere sollte es mit der Vorrichtung und dem Verfahren möglich sein, unterschiedlich große Partikel (z.B. biologische Zellen und/oder Endosomen) einer Flüssigkeit mit einer hohen Trennschärfe voneinander zu trennen und voneinander separierte Partikel auf eine einfache, schnelle und kostengünstige Art und Weise in einer hohen Ausbeute und in einer therapeutisch verwendbaren Flüssigkeit bereitzustellen. Zudem sollten Verwendungen der Vorrichtung vorgeschlagen werden.

Die Aufgabe wird gelöst durch die Vorrichtung mit den Merkmalen von Anspruch 1, das Verfahren mit den Merkmalen von Anspruch 12 und die Verwendung mit den Merkmalen von Anspruch 14. Die abhängigen Ansprüche zeigen vorteilhafte Weiterbildungen auf.

Erfindungsgemäß wird eine Vorrichtung zur Separation von unterschiedlich großen Partikeln in einer Flüssigkeit bereitgestellt, enthaltend
a) einen Behälter zur Aufnahme einer Flüssigkeit;
b) mindestens ein Filterelement mit einer Oberseitenfläche, einer Unterseitenfläche und mindestens einer die Oberseitenfläche mit der Unterseitenfläche verbindende Seitenfläche,

wobei das Filterelement durchgängige Poren mit einem definierten Porendurchmesser aufweist,
wobei das Filterelement in dem Behälter dergestalt angeordnet ist, dass es den Behälter in Richtung Oberseitenfläche des Filterelements in ein oberes Kompartiment und in Richtung Unterseitenfläche des Filterelements in ein unteres Kompartiment aufteilt, sodass Partikel einer Flüssigkeit im oberen Kompartiment nur dann in das untere Kompartiment gelangen können, wenn sie das Filterelement passieren, wobei das obere Kompartiment des Behälters eine Öffnung zur Aufnahme einer Flüssigkeit mit Partikeln aufweist,
wobei die Vorrichtung ein Mittel zur Veränderung des Porendurchmessers der Poren des Filterelements aufweist,
dadurch gekennzeichnet, dass das Mittel zur Veränderung des Porendurchmessers der Poren des Filterelements dazu geeignet ist, eine Kraft auf das Filterelement auszuüben, die entweder vom Zentrum einer Oberfläche des Filterelements in Richtung der Ränder dieser Oberfläche des Filterelements, oder in die entgegengesetzte Richtung, gerichtet ist.

Die erfindungsgemäße Vorrichtung weist den Vorteil auf, dass unterschiedlich große Partikel (z.B. biologische Zellen und/oder Exo-/Endosomen) einer Flüssigkeit mit einer hohen Trennschärfe voneinander getrennt werden können. Die hohe Trennschärfe wird durch das Mittel der Vorrichtung bereitgestellt, das zur Veränderung des Porendurchmessers der Poren des Filterelements geeignet ist. Über dieses Mittel kann der Porendurchmesser im Laufe eines Separationsverfahrens gezielt verändert werden (d.h. insbesondere vergrößert werden), um nacheinander Partikel mit verschiedener Partikelgröße (d.h. insbesondere erst kleinere Partikel und dann größere Partikel) durch das Filterelement passieren zu lassen. Die Vorrichtung ermöglicht damit eine zeitliche Abfolge der Freisetzung der unterschiedlichen Partikel in das untere Kompartiment des Behälters der Vorrichtung. Vor jedem Verändern des Porendurchmessers des Filterelements kann die jeweilige Flüssigkeit mit den passierten Partikeln aus dem unteren Kompartiment entfernt werden, sodass am Ende des Separationsverfahrens mehrere separate Flüssigkeiten vorliegen, die sich darin unterscheiden, dass sie Partikel mit einem unterschiedlichen Partikeldurchmesser enthalten. Die Veränderung des Porendurchmessers ist nicht nur stufenweise, sondern linear möglich, sodass über die Vorrichtung eine sehr hohe Trennschärfe erreicht werden kann.

Ferner können die Partikel der Ausgangsflüssigkeit einfach, schnell und kostengünstig in einer hohen Ausbeute voneinander getrennt werden. Die Einfachheit, Schnelligkeit und geringe Kosten ergeben sich dadurch, dass nur eine einige Vorrichtung zur Separation der Partikel der Flüssigkeit verwendet wird, d.h. nicht mehrere verschiedene Vorrichtungen nacheinander verwendet werden müssen, um die Separation der Partikel zu erreichen. Dies bewirkt auch eine höhere Ausbeute an Partikeln, da die Partikel während ihres Trennverfahrens mit weniger Oberflächen in Kontakt kommen, an die sie sonst adsorbieren können. Es bewirkt auch, dass das Separationsverfahren mit der erfindungsgemäßen Vorrichtung sehr schnell durchführbar ist, da Reinigungsschritte mit weiteren Vorrichtungen entfallen können. Die Vorrichtung ist zudem kostengünstig bereitstellbar, da sie sich aus Komponenten zusammensetzt, die nicht teuer sind. Ein weiterer Vorteil ist die Skalierbarkeit der Vorrichtung, d.h. die Eignung der Vorrichtung, bei entsprechender Vergrößerung des Behälters (insbesondere von dessen oberem Kompartiment) Flüssigkeiten mit einem sehr großen Volumen aufzunehmen und deren Partikel nach Größe zu separieren.

Zudem ist ein Vorteil der Vorrichtung, dass die Partikel nach ihrer Separation in dem löslichen Teil ihrer Ausgangsflüssigkeit vorliegen können. Anders ausgedrückt ist es über die Vorrichtung möglich, dass der lösliche Anteil der Ausgangsflüssigkeit, in der sich die unterschiedlich großen Partikel am Anfang der Separation befinden, am Ende der Separation unverändert bleibt. Im Falle von Blut als Ausgangsflüssigkeit mit Partikeln, d.h. Blutplasma als Ausgangsflüssigkeit mit Blutzellen und Exosomen als Partikeln, ist dies ein entscheidender Vorteil, da die separierten Partikel dann in Blutplasma vorliegen können. Blutplasma stellt eine therapeutisch verwendbare Flüssigkeit dar, da diese beispielsweise für Transfusionen geeignet ist.

Die erfindungsgemäße Vorrichtung ist dadurch gekennzeichnet, dass das Mittel zur Veränderung des Porendurchmessers der Poren des Filterelements dazu geeignet ist, eine Kraft auf das Filterelement auszuüben, die entweder vom Zentrum einer Oberfläche des Filterelements in Richtung der Ränder dieser Oberfläche des Filterelements, oder in die entgegengesetzte Richtung, gerichtet ist. Über eine solche Krafteinwirkung kann der Porendurchmesser der Poren des Filterelements vergrößert bzw. verkleinert werden.

Das Mittel zur Veränderung der Porenweite der Poren des Filterelements kann eine Zentrifuge aufweisen und mindestens einen Körper enthalten, der mit einer Außenseite der mindestens eine Seitenfläche des Filterelements kraftschlüssig verbunden ist, oder mindestens zwei Körper enthält, die jeweils mit einer Außenseite von zwei gegenüberliegende Seitenflächen des Filterelements kraftschlüssig verbunden sind. Der mindestens eine Körper zeichnet sich dadurch aus, dass er dazu geeignet ist, über eine Änderung der Rotationsgeschwindigkeit der Zentrifuge eine Presskraft oder Zugkraft auf die mindestens eine Seitenfläche des Filterelements auszuüben, sodass die Poren des Filterelements komprimiert oder gedehnt werden. Die Wirkweise dieses Mittels beruht daher auf einer Zugwirkung bzw. Presswirkung auf die Seitenfläche des Filterelements, die in Abhängigkeit der Schwerkraft erfolgt, welche wiederrum über die Rotationsgeschwindigkeit der Zentrifuge einstellbar ist. Ein solches Mittel erlaubt eine schnellere und einfachere Einstellung des Porendurchmessers als beispielsweise über ein Mittel, mit dem der Porendurchmesser durch eine mechanisch einstellbare Druckausübung auf das mindestens eine Filterelement erfolgt (z.B. eine mechanisch über Schrauben einer Einspannvorrichtung einstellbare Druckausübung auf das mindestens eine Filterelement).

Ferner kann das Mittel zur Veränderung der Porenweite der Poren des Filterelements eine Zentrifuge aufweisen und mindestens einen Körper, bevorzugt mehrere Körper, enthalten, die an der Oberseite des Filterelements und/oder im Filterelement angeordnet sind und die besonders bevorzugt eine höhere spezifische Dichte und/oder eine höhere elektrische Ladung als die zu trennenden Partikel aufweisen. Der mindestens eine Körper, bevorzugt die mehreren Körper, sind besonders bevorzugt als Nanopartikel ausgestaltet, wobei die Nanopartikel insbesondere um die Poren des Filterelements angeordnet sind. Der mindestens eine Körper zeichnet sich dadurch aus, dass er dazu geeignet ist, über eine Änderung der Rotationsgeschwindigkeit der Zentrifuge eine Presskraft oder Zugkraft auf die Poren des Filterelements auszuüben, sodass die Poren des Filterelements komprimiert oder gedehnt werden. Die Wirkweise dieses Mittels beruht daher auf einer in Abhängigkeit von der Schwerkraft lokal auf die Poren des Filterelements anliegenden Zugwirkung bzw. Presswirkung. Die Stärke der Schwerkraft ist auch hier über die Rotationsgeschwindigkeit der Zentrifuge einstellbar. Ein solches Mittel erlaubt eine schnellere und einfachere Einstellung des Porendurchmessers als beispielsweise über ein Mittel, mit dem der Porendurchmesser durch eine mechanisch einstellbare Druckausübung auf das mindestens eine Filterelement erfolgt (z.B. eine mechanisch über Schrauben einer Einspannvorrichtung einstellbare Druckausübung auf das mindestens eine Filterelement). Vorteil dieser Ausgestaltungsform gegenüber der oben genannten Ausführungsform ist, dass der mindestens eine Körper Teil innerhalb des Behälters angeordnet ist und die Vorrichtung somit kompakter ausgestaltet werden kann als wenn der mindestens eine Körper mit einer Außenseite der mindestens eine Seitenfläche des Filterelements kraftschlüssig verbunden ist.

Abgesehen davon kann das Mittel zur Veränderung der Porenweite der Poren des Filterelements eine elektrische Spannungsquelle aufweist und mindestens eine elektrisch leitfähige Schicht, optional mindestens zwei elektrisch leitfähige Schichten, aufweisen, wobei die elektrische Spanungsquelle mit der mindestens einen elektrisch leitfähigen Schicht, optional mit den mindestens zwei elektrisch leitfähigen Schichten, elektrisch leitend verbunden ist, wobei die mindestens zwei elektrisch leitfähigen Schichten bevorzugt an zwei gegenüberliegenden Flächen der mindestens einen Seitenfläche des Filterelements angeordnet sind und zueinander elektrisch isoliert sind. Die elektrische Spannungsquelle zeichnet sich dadurch aus, dass sie dazu geeignet ist, über eine Änderung ihrer elektrischen Spannung eine Presskraft oder Zugkraft auf die mindestens eine elektrisch leitfähige Schicht, optional die mindestens zwei elektrisch leitfähigen Schichten, auszuüben, sodass die Poren des Filterelements komprimiert oder gedehnt werden. Ein solches Mittel erlaubt eine schnellere und einfachere Einstellung des Porendurchmessers als beispielsweise über ein Mittel, mit dem der Porendurchmesser durch eine mechanisch einstellbare Druckausübung auf das mindestens eine Filterelement erfolgt (z.B. eine mechanisch über Schrauben einer Einspannvorrichtung einstellbare Druckausübung auf das mindestens eine Filterelement). In dieser Ausgestaltungsform kann die Vorrichtung eine Zentrifuge aufweisen. Ein Vorteil hierbei ist, dass die Partikel in der Flüssigkeit schneller als mit bloßer Schwerkraft getrennt werden können, da die Zentrifugalkraft der Zentrifuge das Passieren der Flüssigkeit und Partikel durch das mindestens eine Filterelement beschleunigt. Die elektrische Spannungsquelle kann dazu konfiguriert sein, eine elektrische Spannung im Bereich von 500 bis 4000 V an die mindestens eine elektrisch leitfähige Schicht, optional die mindestens zwei elektrisch leitfähigen Schichten, anzulegen.

In dieser Ausgestaltungsform enthält das Filterelement bevorzugt ein elektroaktives Material und/oder ein piezoelektrisches Material das durchgängige Poren aufweist, oder besteht daraus. Besonders bevorzugt enthält das Material ein elektroaktives Polymer und/oder piezoelektrisches Polymer, insbesondere ein magnetorheologisches Elastomer und/oder piezoelektrisches Elastomer, oder besteht daraus. Hierbei kann es sich um ein Elastomer (beispielsweise ausgewählt aus der Gruppe bestehend aus Silikonelastomer, thermoplastisches Elastomer und Kombinationen hiervon) handeln, das eingebettete magnetische und/oder piezoelektrische Nanopartikel enthält. Unter dem Begriff "Nanopartikel" werden erfindungsgemäß Partikel mit einem Durchmesser von 1 nm bis 100 µm, gemessen durch Elektronenmikrosopie, verstanden. Das erfindungsgemäße Verständnis des Begriffs "Nanopartikel" umfasst somit auch "Mikropartikel", wenn für den Begriff "Mikropartikel" ein Durchmesser von 1 µm bis 100 µm angenommen wird. Die magnetischen und/oder piezoelektrischen Partikel können ausgewählt sein aus der Gruppe bestehend aus Lithiumniobat, Lithiumtantalat und Kombinationen hiervon.

In einer bevorzugten Ausgestaltungsform weist die Vorrichtung eine Steuereinheit auf, die konfiguriert ist, das Mittel zur Veränderung des Porendurchmessers der Poren des Filterelements zu steuern.

Bevorzugt erfolgt die Steuerung dergestalt, dass eine Zentrifugalgeschwindigkeit einer Zentrifuge des Mittels zur Veränderung des Porendurchmessers der Poren des Filterelements geändert wird, bevorzugt dergestalt, dass die Zentrifugalgeschwindigkeit im Laufe der Separation von Partikeln in einer Flüssigkeit stufenweise erhöht wird, wobei die Erhöhung insbesondere automatisch im Laufe der Zeit oder manuell durch Eingabe eines Benutzers erfolgt.

Ferner kann die Steuereinheit konfiguriert sein, das Mittel zur Veränderung des Porendurchmessers der Poren des Filterelements zu dergestalt zu steuern, dass eine elektrische Spannung einer Spannungsquelle des Mittels zur Veränderung des Porendurchmessers der Poren des Filterelements geändert wird, bevorzugt dergestalt, dass die elektrische Spannung im Laufe der Separation von Partikeln in einer Flüssigkeit stufenweise verringert wird, wobei die Verringerung insbesondere automatisch im Laufe der Zeit oder manuell durch Eingabe eines Benutzers erfolgt.

Zudem kann die Steuereinheit konfiguriert sein, das Mittel zur Veränderung des Porendurchmessers der Poren des Filterelements zu dergestalt zu steuern, dass der Porendurchmesser der Poren des Filterelements in einem Bereich von 100 nm bis 100 µm verändert wird. Ein Porendurchmesser in diesem Bereich ist vorteilhaft für die Separation von Blutpartikeln, d.h. die Separation von Blutzellen und Exosomen, die im Blut vorkommen.

Darüber hinaus kann die Steuereinheit konfiguriert sein, das Mittel zur Veränderung des Porendurchmessers der Poren des Filterelements zu dergestalt zu steuern, dass der Porendurchmesser der Poren des Filterelements automatisch im Laufe der Zeit oder manuell durch Eingabe(n) von einem Benutzer der Vorrichtung schrittweise auf einen größeren Porendurchmesser verändert wird, bevorzugt von einem Porendurchmesser von 200 nm bis zu einem Porendurchmesser von 20 µm und größer, besonders bevorzugt von einem Porendurchmesser von 200 nm (vorteilhaft zum Zurückhalten von Exosomen) über einen Porendurchmesser von 3 µm (vorteilhaft zum Zurückhalten von Thrombozyten), einen Porendurchmesser von 4-7 µm (vorteilhaft zum Zurückhalten von Erythrocyten), einem Porendurchmesser von 8-12 µm (vorteilhaft zum Zurückhalten von PBMC), einem Porendurchmesser von 20 µm (vorteilhaft zum Zurückhalten von Makrophagen, Gewebezellen und zirkulierende Tumorzellen) bis zu einem Porendurchmesser von 100 µm (um auch die Makrophagen, Gewebezellen und zirkulierende Tumorzellen, bevorzugt schrittweise, passieren zu lassen).

In einer bevorzugten Ausgestaltungsform enthält die Vorrichtung n weitere Filterelemente, die in Richtung oberes Kompartiment des Behälters auf dem mindestens einen Filterelement angeordnet ist und die jeweils durchgängige Poren mit einem definierten Porendurchmesser aufweisen, wobei der definierte Porendurchmesser der n Filterelemente größer ist als der definierte Porendurchmesser des mindestens einen Filterelements und für jedes der n Filterelemente umso größer ist, je näher sich das jeweilige Filterelement in Richtung des oberen Kompartiments des Behälters befindet, wobei n bevorzugt eine ganze Zahl ≥ 2, besonders bevorzugt eine ganze Zahl ≥ 3, insbesondere eine ganze Zahl im Bereich von 4 bis 10, ist. Die n weiteren Filterelemente können jeweils bis auf den Porendurchmesser der Poren eine, mehrere oder alle Eigenschaften des mindestens einen Filterelements der Vorrichtung aufweisen. Durch die n weiteren Filterelemente entsteht ein sog. "Tiefenfilter", d.h. ein Filter, in dem Partikel der Flüssigkeit je nach ihrer Größe in bestimmten der n Filterelemente festgehalten werden können, d.h. nicht in ein weiter in Richtung unteres Kompartiment angeordnetes Filterelement vordringen können. Der Vorteil an diesem "Tiefenfilter" ist, dass eine Verstopfung der Poren des Filterelements vermieden werden kann.

Die Vorrichtung kann hierbei mindestens ein zweites Filterelement enthalten, das in Richtung oberes Kompartiment des Behälters auf dem Filterelement angeordnet ist und das durchgängige Poren mit einem zweiten, definierten Porendurchmesser aufweist, der größer ist als der Porendurchmesser des Filterelements. Das mindestens eine zweite Filterelement kann bis auf den Porendurchmesser der Poren eine, mehrere oder alle Eigenschaften des mindestens einen Filterelements der Vorrichtung aufweisen. Vor einer Vergrößerung des Porendurchmessers dieser beiden Filterelemente kann beispielsweise eine erste Gruppe von kleineren Partikeln in dem mindestens einen (= unteren) Filterelement angeordnet sein und eine zweite Gruppe von (größeren) Partikeln in dem zweiten (= oberen) Filterelement angeordnet sein. Erst nach einer entsprechenden Vergrößerung des Porendurchmessers können die Partikel der zweiten Gruppe das mindestens eine Filterelement passieren, um letztlich in das untere Kompartiment der Vorrichtung zu gelangen.

Optional kann hierbei die Vorrichtung mindestens ein drittes Filterelement enthalten, das auf einer dem Filterelement abgewandten Seite des zweiten Filterelements angeordnet ist und das durchgängige Poren mit einem dritten, definierten Porendurchmesser aufweist, der größer ist als der Porendurchmesser des zweiten Filterelements. Das mindestens eine dritte Filterelement kann bis auf den Porendurchmesser der Poren eine, mehrere oder alle Eigenschaften des mindestens einen Filterelements der Vorrichtung aufweisen. Durch das zusätzliche dritte Filterelement wird der "Tiefenfilter" verstärkt. Vor einer Vergrößerung des Porendurchmessers dieser drei Filterelemente kann sich eine erste Gruppe von kleinen Partikeln im mindestens einen Filterelement anordnen, eine zweite Gruppe von größeren Partikeln im zweiten Filterelement anordnen und eine dritte Gruppe von noch größeren Partikeln im dritten Filterelement anordnen. Erst nach einer entsprechenden Vergrößerung des Porendurchmessers können die Partikel der zweiten Gruppe das mindestens eine Filterelement passieren und ggf. die Partikel der dritten Gruppe das zweite Filterelement (optional auch das mindestens eine Filterelement) passieren, um letztlich in das untere Kompartiment der Vorrichtung zu gelangen. Der Vorteil, eine Verstopfung der Poren des Filterelements zu vermeiden, ist bei dieser Ausgestaltungsform noch stärker ausgeprägt.

Das mindestens eine Filterelement, bevorzugt jeweils alle Filterelemente der Vorrichtung, enthalten bevorzugt Fasern, die durchgängige Poren aufweisen, oder besteht daraus.

Das mindestens eine Filterelement, bevorzugt jeweils alle Filterelemente der Vorrichtung, enthalten bevorzugt ein elastisches Material, bevorzugt ein elastisches Polymer, das durchgängige Poren aufweist, oder bestehen daraus.

Ferner ist es bevorzugt, dass das mindestens eine Filterelement, bevorzugt jeweils alle Filterelemente der Vorrichtung, ein elektroaktives Material, bevorzugt ein elektroaktives Polymer, das durchgängige Poren aufweist, enthält oder daraus besteht. Der Vorteil hierbei ist, dass der Porendurchmesser des Filterelements über ein Anlegen einer elektrischen Spannung gesteuert werden kann, was eine schnelle und feine Einstellung des Porendurchmessers erlaubt.

Abgesehen davon kann das mindestens eine Filterelement, bevorzugt jeweils alle Filterelemente der Vorrichtung, ein piezoelektrisches Material, das durchgängige Poren aufweist, enthalten oder daraus bestehen. Der Vorteil hierbei ist, dass der Porendurchmesser des Filterelements über ein Anlegen einer elektrischen Spannung gesteuert werden kann, was eine schnelle und feine Einstellung des Porendurchmessers erlaubt.

Das mindestens einen Filterelement, bevorzugt alle Filterelemente der Vorrichtung, kann ein Material enthalten oder daraus bestehen, das ausgewählt ist aus der Gruppe bestehend aus Silikonelastomer, thermoplastisches Elastomer (TPE), magnetorheologisches Elastomer, piezoelektrisches Elastomer, thermoplastisches Urethan (TPU) und Kombinationen hiervon.

Ferner kann das mindestens einen Filterelement, bevorzugt alle Filterelemente der Vorrichtung, einen Verbundwerkstoff enthalten oder daraus bestehen, der bevorzugt ein Elastomer und darin eingebettete (z.B. magnetische, piezoelektrische und/oder gravitationssensitive) Nanopartikel enthält oder daraus besteht. Der Vorteil hierbei ist, dass der Porendurchmesser des Filterelements im Falle von magnetischen und/oder piezoelektrischen Partikeln (Nanopartikeln) über ein Anlegen einer elektrischen Spannung gesteuert werden kann und im Falle von gravitationssensitiven Nanopartikeln über ein Anlegen einer Beschleunigungskraft (z.B. Zentrifugalkraft) gesteuert werden kann. In diesen Fällen ist eine schnelle und feine Einstellung des Porendurchmessers möglich. Unter dem Begriff "gravitationssensitiv" wird erfindungsgemäß insbesondere verstanden, dass die Partikel eine spezifische Dichte von ≥ 2 g/cm³ aufweisen. Unter dem Begriff "Nanopartikel" werden erfindungsgemäß Partikel mit einem Durchmesser von 1 nm bis 100 µm, gemessen durch Elektronenmikrosopie, verstanden (d.h. es sind auch "Mikropartikel" von diesem Verständnis umfasst, wenn für den Begriff "Mikropartikel" ein Durchmesser von 1 µm bis 100 µm angenommen wird). Die gravitationssensitiven Nanopartikel können ausgewählt sein aus der Gruppe bestehend aus Metallpartikel, beschichtete Metallpartikel (z.B. mit einer Keramik beschichtete Metallpartikel), Keramikpartikel und Kombinationen hiervon.

Zudem kann das mindestens eine Filterelement, bevorzugt alle Filterelemente der Vorrichtung, ein Gewebe oder Gewirk aus Fasern enthalten oder daraus bestehen. Das Material des Gewebes und/oder Gewirks muss keine elastischen Eigenschaften (auf molekularer Ebene) aufweisen. Das Material des Gewebes und/oder Gewirks kann ausgewählt sein aus der Gruppe bestehend aus PES, PET, PC, PMMA, COC, Nylon, Glasfasern, PVDF, PP und Kombinationen hiervon.

Abgesehen davon kann das mindestens einen Filterelement, bevorzugt alle Filterelemente der Vorrichtung, ein Material enthalten oder daraus bestehen, das als (asymmetrischer) fester Schaum ausgestaltet ist. Sind mehrere Filterelemente vorhanden, ist es bevorzugt, dass die mehreren Filterelemente einen festen Schaum enthalten oder daraus bestehen, wobei besonders bevorzugt die unterschiedliche Porengrößen der einzelnen Filterelemente innerhalb des festen Schaums stetig ineinander übergehen und somit nur noch theoretische Schichten der einzelnen Filterelemente in dem Schaum existieren. Das Material des festen Schaums kann hierbei ausgewählt sein aus der Gruppe bestehend aus Silikonelastomer, thermoplastisches Elastomer (TPE), magnetorheologisches Elastomer, piezoelektrisches Elastomer, thermoplastisches Urethan (TPU) und Kombinationen hiervon. Ferner kann das Material des festen Schaums einen Verbundwerkstoff enthalten oder daraus bestehen, der bevorzugt ein Elastomer und darin eingebettete (z.B. magnetische, piezoelektrische und/oder gravitationssensitive) Nanopartikel enthält oder daraus besteht. Abgesehen davon kann das Material des festen Schaums ausgewählt sein aus der Gruppe bestehend aus PES, PET, PC, PMMA, COC, Nylon, Glasfasern, PVDF, PP und Kombinationen hiervon.

Darüber hinaus kann das mindestens eine Filterelement, bevorzugt jeweils alle Filterelemente der Vorrichtung, eine Ausdehnung von dessen Oberseite zu dessen Unterseite von > 250 µm, bevorzugt von ≥ 500 µm, besonders bevorzugt von ≥ 1 mm, ganz besonders bevorzugt von ≥ 2 mm, insbesondere im Bereich von 3 mm bis 10 mm, aufweisen.

Das mindestens eine Filterelement, bevorzugt jeweils alle Filterelemente der Vorrichtung, kann eine Beschichtung aufweisen, die dazu geeignet ist, bestimmte Partikel einer Flüssigkeit reversibel zu binden. Bevorzugt enthält oder besteht die Beschichtung aus einem Material, das geeignet ist, durch das Mittel zur Veränderung des Porendurchmessers der Poren des Filterelements so beeinflusst zu werden, dass die Bindung zu den bestimmten Partikeln gelöst wird. Ganz besonders bevorzugt enthält das Material ein elektroaktives Material, insbesondere ein elektroaktives Polymer, oder besteht daraus. Ferner ist die Beschichtung bevorzugt an der Oberseitenfläche, Unterseitenfläche und/oder Poreninnenfläche des mindestens einen Filterelements, besonders bevorzugt an einer Oberseitenfläche, Unterseitenfläche und/oder Poreninnenfläche von allen Filterelementen der Vorrichtung, angeordnet. Der Vorteil an der Beschichtung ist, dass ein größenunabhängiges Zurückhalten bestimmter Partikel und ein selektives, anschließendes Freilassen solcher Partikel (insbesondere Partikel mit einem sehr kleinen Partikeldurchmesser) möglich ist. Dadurch kann die Trennschärfe weiter erhöht werden, insbesondere eine Abtrennung von Partikeln mit sehr kleinen Partikeldurchmessern zuverlässiger erfolgen.

Das untere Kompartiment des Behälters der Vorrichtung kann ein Mittel zur Entnahme von Flüssigkeit aus dem unteren Kompartiment, bevorzugt ein Ventil, besonders bevorzugt ein beschleunigungssensitives Ventil und/oder ein spannungsschaltbares Ventil, aufweisen. Hierbei kann insbesondere eine Steuereinheit der Vorrichtung konfiguriert sein, das Mittel zur Entnahme der Flüssigkeit automatisch im Laufe der Zeit, oder manuell durch Eingabe(n) von einem Benutzer, zu öffnen und zu schließen. Vorteil hierbei ist, dass im Laufe des Separationsverfahrens eine jeweils nach jedem Verändern des Porendurchmessers des Filterelements im unteren Kompartiment vorliegende Flüssigkeit mit Partikeln isoliert werden kann, d.h. aus dem unteren Kompartiment entfernt werden kann. Das Entfernen dieser jeweiligen Flüssigkeiten kann gezielt über Anlegen einer bestimmten Beschleunigung und/oder elektrischen Spannung an das Ventil erfolgen und/oder kann manuell oder automatisch erfolgen. Ein automatisches Entfernen ist für den Benutzer mit weniger Aufwand verbunden und damit komfortabler und weniger fehleranfällig.

Der Behälter der Vorrichtung kann ausgewählt sein aus der Gruppe bestehend aus Zentrifugenröhrchen, Blutentnahmespritze, Blutspendebeutel, Kulturbeutel zur biotechnologischen Herstellung von Medikamenten, Bioreaktor zur biotechnologischen Produktion, Probengefäß, Kulturgefäß und Kombinationen hiervon.

Die Partikel, zu dessen Separation sich die Vorrichtung eignet, können ausgewählt sein aus der Gruppe bestehend aus Vesikel, Viruspartikel und biologischen Zellen, bevorzugt ausgewählt sein aus der Gruppe bestehend aus Vesikel, Viruspartikel und biologische Zellen aus Blut, besonders bevorzugt ausgewählt aus der Gruppe bestehend aus endosomalen Vesikeln, exosomale Vesikel (Exosomen), Viruspartikel, Liposomen, Thrombocyten, Erythrocyten, Leukocyten und Kombinationen hiervon.

Ferner wird erfindungsgemäß ein Verfahren zur Separation von unterschiedlich großen Partikeln in einer Flüssigkeit bereitgestellt, umfassend die Schritte
a) Bereitstellen einer erfindungsgemäßen Vorrichtung;
b) Einstellen des Porendurchmessers der Poren des Filterelements der Vorrichtung, sodass entweder keine Partikel oder nur Partikel bis zu einem gewünschten Partikeldurchmesser das Filterelement passieren können;
c) Befüllen des oberen Kompartiments des Behälters der Vorrichtung mit einer Flüssigkeit, die Partikel mit einer unterschiedlichen Größe enthält;
d) Bewegen der Flüssigkeit durch das Filterelement, bevorzugt über ein Mittel ausgewählt aus der Gruppe bestehend aus Zentrifuge, Pumpe und Kombinationen hiervon;
e) Isolieren der Flüssigkeit, die optional passierte Partikel enthält, aus dem unteren Kompartiment des Behälters der Vorrichtung;
f) Erhöhung des Porendurchmessers der Poren des Filterelements der Vorrichtung, sodass Partikel bis zu einem gewünschten, nun größeren Porendurchmesser das Filterelement passieren können;
g) Optional Befüllen des oberen Kompartiments des Behälters der Vorrichtung mit einer Flüssigkeit, die bevorzugt keine Partikel enthält;
h) Bewegen der Flüssigkeit durch das Filterelement, bevorzugt über ein Mittel ausgewählt aus der Gruppe bestehend aus Zentrifuge, Pumpe und Kombinationen hiervon;
i) Isolieren der Flüssigkeit mit den nun passierten Partikeln aus dem unteren Kompartiment des Behälters der Vorrichtung;
j) Optional Aufheben einer reversiblen Bindung von Partikeln an eine Beschichtung mindestens eines Filterelements der Vorrichtung, bevorzugt über einen Einfluss des Mittels zur Veränderung des Porendurchmessers der Poren des Filterelements;
k) Optional Wiederholen der Schritte g) bis j), bis alle Partikel der Flüssigkeit nach ihrer Größe separiert in getrennten Flüssigkeiten vorliegen.

Das Verfahren kann dadurch gekennzeichnet, dass das Erhöhen des Porendurchmessers der Poren des Filterelements durch eine Erhöhung einer Zentrifugalgeschwindigkeit einer Zentrifuge des Mittels zur Veränderung des Porendurchmessers der Poren des Filterelements erfolgt.

Ferner kann das Verfahren dadurch gekennzeichnet sein, dass das Erhöhen des Porendurchmessers der Poren des Filterelements Verringerung einer elektrischen Spannung einer Spannungsquelle des Mittels zur Veränderung des Porendurchmessers der Poren des Filterelements erfolgt.

Zudem wird erfindungsgemäß eine Verwendung der erfindungsgemäßen Vorrichtung zur Separation von Partikeln unterschiedlicher Größe, die in einer Flüssigkeit vorliegen, vorgeschlagen. Die Verwendung der Vorrichtung kann eine Isolation von einer oder mehreren Blutzellfraktionen aus Blut, bevorzugt zur Bereitstellung der Blutzellfraktionen für die Diagnostik und/oder zur Herstellung von Blutpräparaten, insbesondere zur Erzeugung von Zelltherapeutika, sein. Ferner kann die Verwendung der Vorrichtung eine Isolation von Bakterienzellen aus Blut sein. Zudem kann die Verwendung der Vorrichtung eine Isolation von Exosomen aus Blut, Blutserum oder Biosuspensionen, bevorzugt zur Bereitstellung von Exosomen für die Diagnostik und/oder zur Herstellung von Impfstoffen (z.B. Liposomen-Impfstoffen), sein. Abgesehen davon kann die Verwendung der Vorrichtung eine Isolation von Gewebezellen aus gemischten Gewebezellfraktionen sein. Darüber hinaus kann die Verwendung der Vorrichtung eine Isolation von Zellen aus gemischten Zellsuspensionen, die von Bioreaktoren stammen, sein, wobei die Zellen bevorzugt ausgewählt sind aus der Gruppe bestehend aus Pflanzenzellen, tierischen Zellen, menschlichen Zellen, Bakterienzellen, Hefezellen und Kombinationen hiervon.

Anhand der nachfolgenden Figuren und Beispiele soll der erfindungsgemäße Gegenstand näher erläutert werden, ohne diesen auf die hier dargestellten, spezifischen Ausführungsformen einschränken zu wollen.

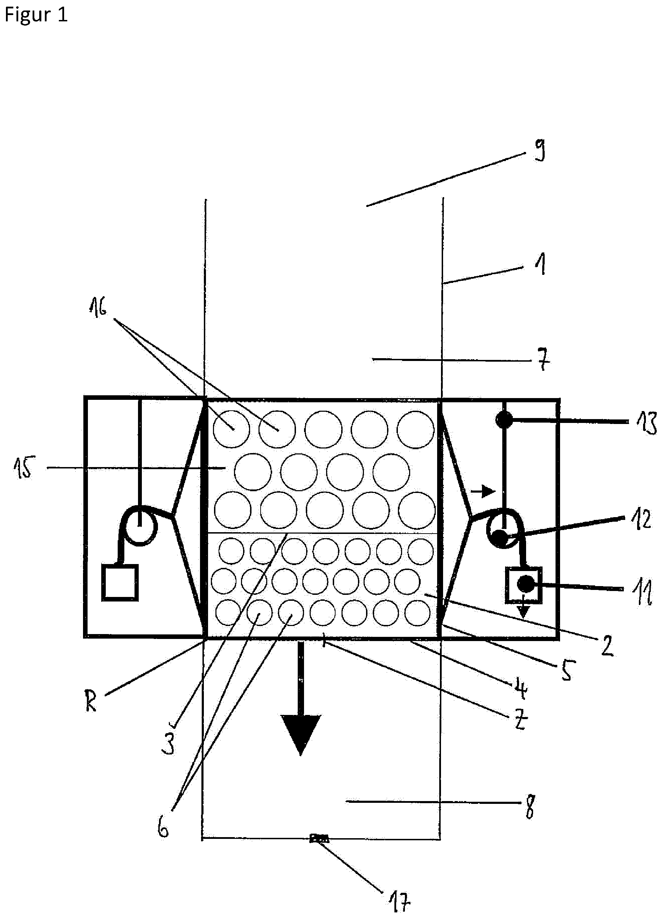

Figur 1 zeigt eine erste Ausgestaltungsform einer erfindungsgemäßen Vorrichtung. Die Vorrichtung enthält einen Behälter 1 zur Aufnahme einer Flüssigkeit und mindestens ein Filterelement 2 mit einer Oberseitenfläche 3, einer Unterseitenfläche 4 und mindestens einer die Oberseitenfläche 3 mit der Unterseitenfläche 4 verbindende Seitenfläche 5. Das Filterelement 2 weist durchgängige Poren 6 mit einem definierten Porendurchmesser auf. Das Filterelement 2 ist in dem Behälter 1 dergestalt angeordnet, dass es den Behälter 1 in Richtung Oberseitenfläche 3 des Filterelements 2 in ein oberes Kompartiment 7 und in Richtung Unterseitenfläche 4 des Filterelements 2 in ein unteres Kompartiment 8 aufteilt, sodass Partikel einer Flüssigkeit im oberen Kompartiment 7 nur dann in das untere Kompartiment 8 gelangen können, wenn sie das Filterelement 2 passieren. Das obere Kompartiment 7 des Behälters 1 weist eine Öffnung 9 zur Aufnahme einer Flüssigkeit mit Partikeln auf. Die Vorrichtung ist dadurch gekennzeichnet, dass sie ein Mittel zur Veränderung des Porendurchmessers der Poren 6 des Filterelements 2 aufweist. Dieses Mittel weist in dieser Ausgestaltungsform eine Zentrifuge (nicht dargestellt) und einen Körper 11 zur Ausübung einer Presskraft oder Zugkraft auf die Seitenwand 5 des Filterelements 2 auf, wobei der Körper 11 hier über eine Schnur kraftschlüssig mit einer Außenseite der mindestens eine Seitenfläche 5 des Filterelements 2 verbunden ist, die über eine an einer Befestigung 13 angebrachten Umlenkrolle 12 abgelenkt wird. Eine stärker werdende Zentrifugalkraft der Zentrifuge bewirkt eine stärkere Kraft auf den Körper 11 und damit eine stärkere Zugkraft auf die Seitenwand 5 des Filterelements 2, wodurch sich die Poren 6 des Filterelements 2 weiten.

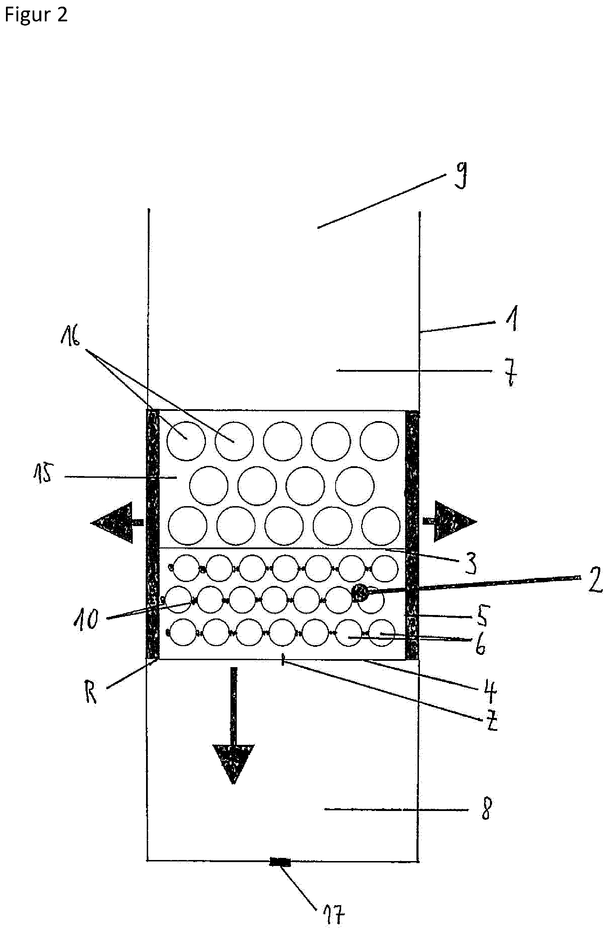

Figur 2 zeigt eine zweite Ausgestaltungsform einer erfindungsgemäßen Vorrichtung. Die Vorrichtung enthält einen Behälter 1 zur Aufnahme einer Flüssigkeit und mindestens ein Filterelement 2 mit einer Oberseitenfläche 3, einer Unterseitenfläche 4 und mindestens einer die Oberseitenfläche 3 mit der Unterseitenfläche 4 verbindende Seitenfläche 5. Das Filterelement 2 weist durchgängige Poren 6 mit einem definierten Porendurchmesser auf. Das Filterelement 2 ist in dem Behälter 1 dergestalt angeordnet, dass es den Behälter 1 in Richtung Oberseitenfläche 3 des Filterelements 2 in ein oberes Kompartiment 7 und in Richtung Unterseitenfläche 4 des Filterelements 2 in ein unteres Kompartiment 8 aufteilt, sodass Partikel einer Flüssigkeit im oberen Kompartiment 7 nur dann in das untere Kompartiment 8 gelangen können, wenn sie das Filterelement 2 passieren. Das obere Kompartiment 7 des Behälters 1 weist eine Öffnung 9 zur Aufnahme einer Flüssigkeit mit Partikeln auf. Die Vorrichtung ist dadurch gekennzeichnet, dass sie ein Mittel zur Veränderung des Porendurchmessers der Poren 6 des Filterelements 2 aufweist. Dieses Mittel weist in dieser Ausgestaltungsform eine Zentrifuge (nicht dargestellt) und mindestens zwei Körper 10 zur Ausübung einer Presskraft oder Zugkraft auf die Poren des Filterelements auf, wobei die mindestens zwei Körper 10 hier als Nanopartikel ausgestaltet sind, die an gegenüberliegenden Seiten der Poren des Filterelements angeordnet sind. Eine stärker werdende Zentrifugalkraft der Zentrifuge bewirkt eine stärkere Kraft auf die mindestens zwei Körper 10 und damit eine stärkere Zugkraft auf die Seitenränder der Poren 6 des Filterelements 2, wodurch sich die Poren 6 des Filterelements 2 weiten.

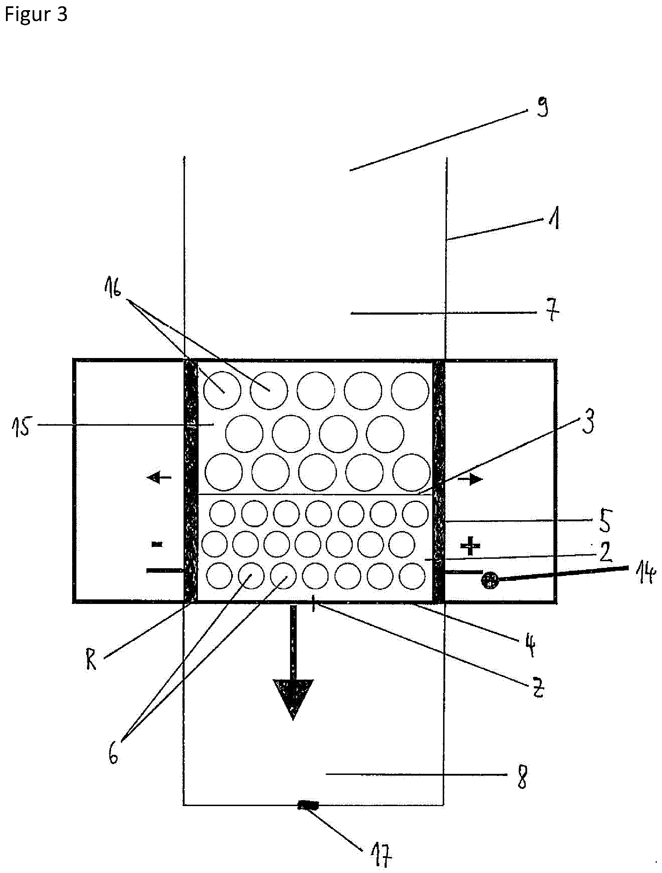

Figur 3 zeigt eine dritte Ausgestaltungsform einer erfindungsgemäßen Vorrichtung. Die Vorrichtung enthält einen Behälter 1 zur Aufnahme einer Flüssigkeit und mindestens ein Filterelement 2 mit einer Oberseitenfläche 3, einer Unterseitenfläche 4 und mindestens einer die Oberseitenfläche 3 mit der Unterseitenfläche 4 verbindende Seitenfläche 5. Das Filterelement 2 weist durchgängige Poren 6 mit einem definierten Porendurchmesser auf. Das Filterelement 2 ist in dem Behälter 1 dergestalt angeordnet, dass es den Behälter 1 in Richtung Oberseitenfläche 3 des Filterelements 2 in ein oberes Kompartiment 7 und in Richtung Unterseitenfläche 4 des Filterelements 2 in ein unteres Kompartiment 8 aufteilt, sodass Partikel einer Flüssigkeit im oberen Kompartiment 7 nur dann in das untere Kompartiment 8 gelangen können, wenn sie das Filterelement 2 passieren. Das obere Kompartiment 7 des Behälters 1 weist eine Öffnung 9 zur Aufnahme einer Flüssigkeit mit Partikeln auf. Die Vorrichtung ist dadurch gekennzeichnet, dass sie ein Mittel zur Veränderung des Porendurchmessers der Poren 6 des Filterelements 2 aufweist. Dieses Mittel weist in dieser Ausgestaltungsform eine elektrische Spannungsquelle 14 und mindestens eine elektrisch leitfähige Schicht an der Seitenwand 5 des Filterelements 2 auf. Eine geringer werdende elektrische Spannung der elektrischen Spannungsquelle bewirkt eine geringere Presskraft auf die Seitenwand 5 des Filterelements 2, wodurch sich die Poren 6 des Filterelements 2 weiten.

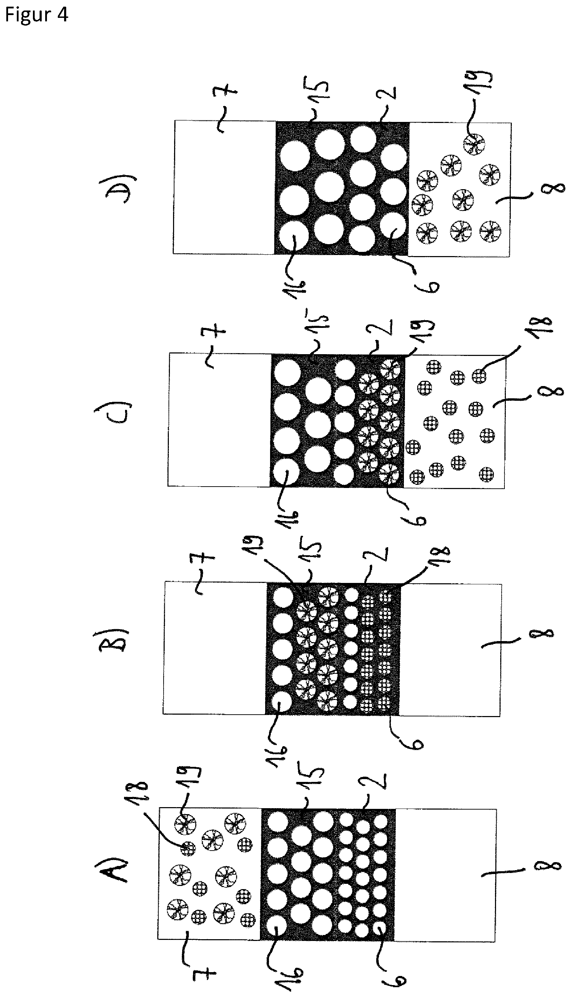

Figur 4 illustriert schematisch die Separation von unterschiedlich großen Partikeln in einer Flüssigkeit über die erfindungsgemäße Vorrichtung, wobei das Mittel zur Veränderung der Porenweite der Poren des Filterelements hier eine elektrische Spannungsquelle aufweist, die mit mindestens einer elektrisch leitfähigen Schicht an der Seitenfläche des Filterelements verbunden ist. An die elektrisch leitfähige Schicht wird keine elektrische Spannung oder nur eine niedrige elektrische Spannung angelegt, um den mittleren Porendurchmesser der Poren 6 des ersten Filterelements 2 und der Poren 16 des zweiten Filterelements 15 möglichst klein zu halten. In das obere Kompartiment 7 des Behälters der Vorrichtung wird eine Flüssigkeit (Suspension) gegeben, die eine erste Gruppe von Partikeln 18 mit einer ersten Größe und eine zweite Gruppe von Partikeln 19 mit einer zweiten Größe enthält, wobei die zweite Größe größer ist als die erste Größe (Figur 4A). Über Schwerkraft oder über die Wirkung einer Zentrifuge und/oder Pumpe passiert einerseits die erste Gruppe von Partikeln 18 das zweite Filterelement 15 und dringt bis in das erste Filterelement 2 vor und wird dort gehalten, wobei die zweite Gruppe von Partikeln 19 nicht in das erste Filterelement 2 vordringen kann und bereits durch das zweite Filterelement 15 zurückgehalten wird (Figur 4B). Wird nun die von der elektrischen Spannungsquelle angelegte Spannung erhöht, weiten sich die Poren 6 des ersten Filterelements 2 und die Poren 15 des zweiten Filterelements 15, sodass die erste Gruppe von Partikeln 18 das erste Filterelement 2 passieren kann und in dem unteren Kompartiment 8 des Behälters der Vorrichtung aufgefangen wird und die zweite Gruppe von Partikeln 19 in das erste Filterelement 2 vordringen kann und dort zurückgehalten wird (Figur 4C). Nach Entfernen der Flüssigkeit mit der ersten Gruppe von Partikeln 18 wird die von der elektrischen Spannungsquelle angelegte Spannung weiter erhöht, sodass sich die Poren 6 des ersten Filterelements 2 noch stärker weiten und nun auch die zweite Gruppe von Partikeln 19 das erste Filterelement 2 passieren kann und in dem unteren Kompartiment 8 des Behälters der Vorrichtung aufgefangen wird (Figur 4D). Dadurch ist eine stufenweise Trennung der ersten Gruppe von Partikeln 18 von der zweiten Gruppe von Partikeln 19 möglich.

### Beispiel 1 - Vorrichtung mit einer Zentrifuge und mindestens einem Körper zur Veränderung des Porendurchmessers

In einem ersten Selektionsschritt, gekennzeichnet durch eine erste Zentrifugationsbeschleunigung, läuft die Flüssigkeit mit Partikeln, die die Poren des Filterelements bei der ersten Zentrifugalbeschleunigung passieren können, vom oberen in das untere Kompartiment des Behälters, während Flüssigkeit mit Partikeln, die die Poren des Filterelements unter diesen Bedingungen nicht passieren können, in dem oberen Kompartiment des Behälters verbleibt. Die Flüssigkeit mit Partikeln, die sich dann im unteren Kompartiment befindet, wird aus dem unteren Kompartiment entfernt.

In einem zweiten Selektionsschritt wird eine zweite Zentrifugalbeschleunigung angelegt, die höher ist als die erste Zentrifugalbeschleunigung, wodurch über die Wirkung des mindestens einen Körpers eine Vergrößerung des Porendurchmessers der Poren des Filterelements bewirkt wird. Dadurch kann nun Flüssigkeit mit größeren Partikeln das Filterelement passierend und in dem unteren Kompartiment des Behälters aufgefangen werden und aus diesem isoliert werden.

### Beispiel 2 - Vorrichtung mit einer elektrischen Spannungsquelle zur Veränderung des Porendurchmessers

In einem ersten Selektionsschritt, gekennzeichnet durch eine erste elektrische Spannung, die über die elektrische Spannungsquelle angelegt wird, läuft die Flüssigkeit mit Partikeln, die die Poren des Filterelements bei der elektrischen Spannung passieren können, vom oberen in das untere Kompartiment des Behälters, während Flüssigkeit mit Partikeln, die die Poren des Filterelements unter diesen Bedingungen nicht passieren können, in dem oberen Kompartiment des Behälters verbleibt. Die Flüssigkeit mit Partikeln, die sich dann im unteren Kompartiment befindet, wird aus dem unteren Kompartiment entfernt.

In einem zweiten Selektionsschritt wird eine elektrische Spannung angelegt, die geringer ist als die erste elektrische Spannung, wodurch eine Vergrößerung des Porendurchmessers der Poren des Filterelements bewirkt wird. Dadurch kann nun Flüssigkeit mit größeren Partikeln das Filterelement passierend und in dem unteren Kompartiment des Behälters aufgefangen werden und aus diesem isoliert werden.

### Bezugszeichenliste

- 1:: Behälter;
- 2:: Filterelement;
- 3:: Oberseite des Filterelements;
- 4:: Unterseite des Filterelements;
- 5:: Seitenfläche des Filterelements;
- 6:: Durchgängige Poren des Filterelements mit definiertem Porendurchmesser;
- 7:: oberes Kompartiment des Behälters;
- 8:: unteres Kompartiment des Behälters;
- 9:: Öffnung des oberen Kompartiments des Behälters;
- 10:: Körper zur Ausübung einer Presskraft oder Zugkraft auf die Poren des Filterelements;
- 11:: Körper zur Ausübung einer Presskraft oder Zugkraft auf die Seitenwand des Filterelements;
- 12:: Umlenkrolle;
- 13:: Befestigung der Umlenkrolle;
- 14:: elektrische Spannungsquelle;
- 15:: zweites Filterelement;
- 16:: Durchgängige Poren des zweiten Filterelements mit definiertem Porendurchmesser;
- 17:: Mittel zur Entnahme von Flüssigkeit aus dem unteren Kompartiment (z.B. Ventil);
- 18:: erste Gruppe von Partikeln;
- 19:: zweite Gruppe von Partikeln, die größer sind als die erste Gruppe von Partikeln;
- Z:: Zentrum einer Oberfläche des Filterelements;
- R:: Rand einer Oberfläche des Filterelements.

## Patentansprüche

1. Vorrichtung zur Separation von unterschiedlich großen Partikeln (18, 19) in einer Flüssigkeit, enthaltend
a) einen Behälter (1) zur Aufnahme einer Flüssigkeit;
b) mindestens ein Filterelement (2) mit einer Oberseitenfläche (3), einer Unterseitenfläche (4) und mindestens einer die Oberseitenfläche (3) mit der Unterseitenfläche (4) verbindende Seitenfläche (5),
wobei das Filterelement (2) durchgängige Poren (6) mit einem definierten Porendurchmesser aufweist,
wobei das Filterelement (2) in dem Behälter (1) dergestalt angeordnet ist, dass es den Behälter (1) in Richtung Oberseitenfläche (3) des Filterelements (2) in ein oberes Kompartiment (7) und in Richtung Unterseitenfläche (4) des Filterelements (2) in ein unteres Kompartiment (8) aufteilt, sodass Partikel (18, 19) einer Flüssigkeit im oberen Kompartiment (7) nur dann in das untere Kompartiment (8) gelangen können, wenn sie das Filterelement (2) passieren, wobei das obere Kompartiment (7) des Behälters (1) eine Öffnung (9) zur Aufnahme einer Flüssigkeit mit Partikeln (18, 19) aufweist,
wobei die Vorrichtung ein Mittel (10, 11, 14) zur Veränderung des Porendurchmessers der Poren (6) des Filterelements (2) aufweist,
**dadurch gekennzeichnet, dass** das Mittel (10, 11, 14) zur Veränderung des Porendurchmessers der Poren (6) des Filterelements (2) dazu geeignet ist, eine Kraft auf das Filterelement (2) auszuüben, die entweder vom Zentrum (Z) einer Oberfläche des Filterelements (2) in Richtung der Ränder dieser Oberfläche des Filterelements (2), oder in die entgegengesetzte Richtung, gerichtet ist.

2. Vorrichtung gemäß dem vorhergehenden Anspruch, **dadurch gekennzeichnet, dass** das Mittel (10, 11, 14) zur Veränderung der Porenweite der Poren (6) des Filterelements (2)
i) eine Zentrifuge aufweist; und
ii) mindestens einen Körper (11) enthält, der mit einer Außenseite der mindestens eine Seitenfläche (5) des Filterelements (2) kraftschlüssig verbunden ist, optional mindestens zwei Körper (11) enthält, die jeweils mit einer Außenseite von zwei gegenüberliegende Seitenflächen (5) des Filterelements (2) kraftschlüssig verbunden sind;
wobei der mindestens eine Körper (11) dazu geeignet ist, über eine Änderung der Rotationsgeschwindigkeit der Zentrifuge eine Presskraft oder Zugkraft auf die mindestens eine Seitenfläche (5) des Filterelements (2) auszuüben, sodass die Poren (6) des Filterelements (2) komprimiert oder gedehnt werden.

3. Vorrichtung gemäß einem der vorhergehenden Ansprüche, **dadurch gekennzeichnet, dass** das Mittel (10, 11, 14) zur Veränderung der Porenweite der Poren (6) des Filterelements (2)
i) eine Zentrifuge aufweist; und
ii) mindestens einen Körper (10), bevorzugt mehrere Körper (10), enthält, die an der Oberseite (3) des Filterelements (2) und/oder im Filterelement (2) angeordnet sind und die besonders bevorzugt eine höhere spezifische Dichte und/oder eine höhere elektrische Ladung als die zu trennenden Partikel (18, 19) aufweisen, wobei ganz besonders bevorzugt der mindestens eine Körper (10), bevorzugt die mehreren Körper (10), als Nanopartikel ausgestaltet sind und die Nanopartikel insbesondere um die Poren (6) des Filterelements (2) angeordnet sind;
wobei der mindestens eine Körper (10) dazu geeignet ist, über eine Änderung der Rotationsgeschwindigkeit der Zentrifuge eine Presskraft oder Zugkraft auf die Poren (6) des Filterelements (2) auszuüben, sodass die Poren (6) des Filterelements (2) komprimiert oder gedehnt werden.

4. Vorrichtung gemäß einem der vorhergehenden Ansprüche, **dadurch gekennzeichnet, dass** das Mittel (10, 11, 14) zur Veränderung der Porenweite der Poren (6) des Filterelements (2)
i) eine elektrische Spannungsquelle (14) aufweist; und
ii) mindestens eine elektrisch leitfähige Schicht, optional mindestens zwei elektrisch leitfähige Schichten, aufweist, wobei die elektrische Spanungsquelle mit der mindestens einen elektrisch leitfähigen Schicht, optional mit den mindestens zwei elektrisch leitfähigen Schichten, elektrisch leitend verbunden ist, wobei die mindestens eine elektrisch leitfähige Schicht an der Seitenfläche (5) des Filterelements (2) angeordnet ist, bevorzugt die zwei elektrisch leitfähigen Schichten an zwei gegenüberliegenden Flächen der mindestens einen Seitenfläche (5) des Filterelements (2) angeordnet sind und zueinander elektrisch isoliert sind,
wobei die elektrische Spannungsquelle (14) dazu geeignet ist, über eine Änderung ihrer elektrischen Spannung eine Presskraft oder Zugkraft auf die mindestens eine elektrisch leitfähige Schicht, optional die mindestens zwei elektrisch leitfähigen Schichten, auszuüben, sodass die Poren (6) des Filterelements (2) komprimiert oder gedehnt werden.

5. Vorrichtung gemäß einem der vorhergehenden Ansprüche, **dadurch gekennzeichnet, dass** die Vorrichtung eine Steuereinheit aufweist, die konfiguriert ist, das Mittel (10, 11, 14) zur Veränderung des Porendurchmessers der Poren (6) des Filterelements (2) zu steuern, bevorzugt dergestalt, dass
i) eine Zentrifugalgeschwindigkeit einer Zentrifuge des Mittels (10, 11) zur Veränderung des Porendurchmessers der Poren (6) des Filterelements (2) geändert wird, bevorzugt dergestalt, dass die Zentrifugalgeschwindigkeit im Laufe der Separation von Partikeln (18, 19) in einer Flüssigkeit stufenweise erhöht wird, wobei die Erhöhung insbesondere automatisch im Laufe der Zeit oder manuell durch Eingabe eines Benutzers erfolgt; und/oder
ii) eine elektrische Spannung einer Spannungsquelle (14) des Mittels (14) zur Veränderung des Porendurchmessers der Poren (6) des Filterelements (2) geändert wird, bevorzugt dergestalt, dass die elektrische Spannung im Laufe der Separation von Partikeln (18, 19) in einer Flüssigkeit stufenweise verringert wird, wobei die Verringerung insbesondere automatisch im Laufe der Zeit oder manuell durch Eingabe eines Benutzers erfolgt; und/oder
iii) der Porendurchmesser der Poren (6) des Filterelements (2) in einem Bereich von 100 nm bis 100 µm verändert wird; und/oder
iv) der Porendurchmesser der Poren (6) des Filterelements (2) automatisch im Laufe der Zeit oder manuell durch Eingabe(n) von einem Benutzer der Vorrichtung schrittweise auf einen größeren Porendurchmesser verändert wird, bevorzugt von einem Porendurchmesser von 200 nm bis zu einem Porendurchmesser von 20 µm und größer, besonders bevorzugt von einem Porendurchmesser von 200 nm über einen Porendurchmesser von 3 µm, einen Porendurchmesser von 7 µm, einem Porendurchmesser von 8-12 µm, einem Porendurchmesser von 20 µm bis zu einem Porendurchmesser von 100 µm.

6. Vorrichtung gemäß einem der vorhergehenden Ansprüche, **dadurch gekennzeichnet, dass** die Vorrichtung n weitere Filterelemente (15) enthält, die in Richtung oberes Kompartiment (7) des Behälters (1) auf dem mindestens einen Filterelement (2) angeordnet ist und die jeweils durchgängige Poren (16) mit einem definierten Porendurchmesser aufweisen, wobei der definierte Porendurchmesser der n Filterelemente (15) größer ist als der definierte Porendurchmesser des mindestens einen Filterelements (2) und für jedes der n Filterelemente (15) umso größer ist, je näher sich das jeweilige Filterelement (15) in Richtung des oberen Kompartiments (8) des Behälters (1) befindet, wobei n bevorzugt eine ganze Zahl ≥ 2, besonders bevorzugt eine ganze Zahl ≥ 3, insbesondere eine ganze Zahl im Bereich von 4 bis 10, ist.

7. Vorrichtung gemäß einem der vorhergehenden Ansprüche, **dadurch gekennzeichnet, dass** das mindestens eine Filterelement (2), bevorzugt jeweils alle Filterelemente (2, 15) der Vorrichtung,
i) Fasern, die durchgängige Poren (6, 16) aufweisen, enthält oder daraus besteht; und/oder
ii) ein elastisches Material, bevorzugt ein elastisches Polymer, das durchgängige Poren (6, 16) aufweist, enthält oder daraus besteht; und/oder
iii) ein elektroaktives Material, bevorzugt ein elektroaktives Polymer, das durchgängige Poren (6, 16) aufweist, enthält oder daraus besteht; und/oder
iv) ein piezoelektrisches Material, bevorzugt ein piezoelektrisches Polymer, das durchgängige Poren (6, 16) aufweist, enthält oder daraus besteht; und/oder
v) ein Material enthält oder daraus besteht, das ausgewählt ist aus der Gruppe bestehend aus Silikonelastomer, thermoplastisches Elastomer, magnetorheologisches Elastomer, piezoelektrisches Elastomer, thermoplastisches Urethan und Kombinationen hiervon; und/oder
vi) einen Verbundwerkstoff enthält oder daraus besteht, wobei der Verbundwerkstoff bevorzugt ein Elastomer und darin eingebettete Nanopartikel enthält oder daraus besteht, wobei die Nanopartikel bevorzugt magnetische, piezoelektrische und/oder gravitationssensitive Nanopartikel sind; und/oder
vii) ein Fasergewebe oder Fasergewirk enthält oder daraus besteht, wobei das Fasergewebe oder Fasergewirk bevorzugt ein Material enthält oder daraus besteht, das ausgewählt ist aus der Gruppe bestehend aus PES, PET, PC, PMMA, COC, Nylon, Glasfasern, PVDF, PP und Kombinationen hiervon; und/oder
viii) einen festen Schaum enthält oder daraus besteht, wobei der Schaum bevorzugt ein Material enthält oder daraus besteht, das ausgewählt ist aus der Gruppe bestehend aus Silikonelastomer, thermoplastisches Elastomer, magnetorheologisches Elastomer, piezoelektrisches Elastomer, thermoplastisches Urethan, Verbundwerkstoff aus einem Elastomer mit darin eingebetteten Nanopartikeln, PES, PET, PC, PMMA, COC, Nylon, Glasfasern, PVDF, PP und Kombinationen hiervon; und/oder
ix) eine Ausdehnung von dessen Oberseite (3) zu dessen Unterseite (4) von > 250 µm, bevorzugt von ≥ 500 µm, besonders bevorzugt von ≥ 1 mm, ganz besonders bevorzugt von ≥ 2 mm, insbesondere im Bereich von 3 mm bis 10 mm, aufweist.

8. Vorrichtung gemäß einem der vorhergehenden Ansprüche, **dadurch gekennzeichnet, dass** das mindestens eine Filterelement (2), bevorzugt jeweils alle Filterelemente (2, 15) der Vorrichtung, eine Beschichtung aufweist, die dazu geeignet ist, bestimmte Partikel (18, 19) einer Flüssigkeit reversibel zu binden, wobei die Beschichtung bevorzugt
i) ein Material enthält oder daraus besteht, das geeignet ist, durch das Mittel (10, 11, 14) zur Veränderung des Porendurchmessers der Poren (6, 16) des Filterelements (2) so beeinflusst zu werden, dass die Bindung zu den bestimmten Partikeln (18, 19) gelöst wird, wobei das Material ganz besonders bevorzugt ein elektroaktives Material, insbesondere ein elektroaktives Polymer, enthält oder daraus besteht; und/oder
ii) an der Oberseitenfläche (3), Unterseitenfläche (4) und/oder Poreninnenfläche des mindestens einen Filterelements (2), besonders bevorzugt an einer Oberseitenfläche (3), Unterseitenfläche (4) und/oder Poreninnenfläche von allen Filterelementen (2) der Vorrichtung, angeordnet ist.

9. Vorrichtung gemäß einem der vorhergehenden Ansprüche, **dadurch gekennzeichnet, dass** das untere Kompartiment (8) des Behälters (1) der Vorrichtung ein Mittel (17) zur Entnahme von Flüssigkeit aus dem unteren Kompartiment (8), bevorzugt ein Ventil, besonders bevorzugt ein beschleunigungssensitives Ventil und/oder ein spannungsschaltbares Ventil, aufweist, wobei insbesondere eine Steuereinheit der Vorrichtung konfiguriert ist, das Mittel (17) zur Entnahme der Flüssigkeit automatisch im Laufe der Zeit, oder manuell durch Eingabe(n) von einem Benutzer, zu öffnen und zu schließen.

10. Vorrichtung gemäß einem der vorhergehenden Ansprüche, **dadurch gekennzeichnet, dass** der Behälter (1) ausgewählt ist aus der Gruppe bestehend aus Zentrifugenröhrchen, Blutentnahmespritze, Blutspendebeutel, Kulturbeutel zur biotechnologischen Herstellung von Medikamenten, Bioreaktor zur biotechnologischen Produktion, Probengefäß, Kulturgefäß und Kombinationen hiervon.

11. Vorrichtung gemäß einem der vorhergehenden Ansprüche, **dadurch gekennzeichnet, dass** die Partikel (18, 19) ausgewählt sind aus der Gruppe bestehend aus Vesikel, Viruspartikeln und biologischen Zellen; bevorzugt ausgewählt aus der Gruppe bestehend aus Vesikel, Viruspartikel und biologische Zellen aus Blut, besonders bevorzugt ausgewählt aus der Gruppe bestehend aus endosomalen Vesikeln, exosomalen Vesikeln, Viruspartikeln, Liposomen, Thrombocyten, Erythrocyten, Leukocyten und Kombinationen hiervon.

12. Verfahren zur Separation von unterschiedlich großen Partikeln (18, 19) in einer Flüssigkeit, umfassend die Schritte
a) Bereitstellen einer Vorrichtung gemäß einem der Ansprüche 1 bis 11;
b) Einstellen des Porendurchmessers der Poren (6, 16) des Filterelements (2, 15) der Vorrichtung, sodass entweder keine Partikel (18, 19) oder nur Partikel (18) bis zu einem gewünschten Partikeldurchmesser das Filterelement (2, 15) passieren können;
c) Befüllen des oberen Kompartiments des Behälters (1) der Vorrichtung mit einer Flüssigkeit, die Partikel (18, 19) mit einer unterschiedlichen Größe enthält;
d) Bewegen der Flüssigkeit durch das Filterelement (2, 15), bevorzugt über ein Mittel ausgewählt aus der Gruppe bestehend aus Zentrifuge, Pumpe und Kombinationen hiervon;
e) Isolieren der Flüssigkeit, die optional passierte Partikel (18) enthält, aus dem unteren Kompartiment (8) des Behälters (1) der Vorrichtung;
f) Erhöhung des Porendurchmessers der Poren (6, 16) des Filterelements (2) der Vorrichtung, sodass Partikel (19) bis zu einem gewünschten Porendurchmesser das Filterelement (2, 15) passieren können;
g) Optional Befüllen des oberen Kompartiments des Behälters (1) der Vorrichtung mit einer Flüssigkeit, die bevorzugt keine Partikel (18, 19) enthält;
h) Bewegen der Flüssigkeit durch das Filterelement (2, 15), bevorzugt über ein Mittel ausgewählt aus der Gruppe bestehend aus Zentrifuge, Pumpe und Kombinationen hiervon;
i) Isolieren der Flüssigkeit mit den passierten Partikeln (19) aus dem unteren Kompartiment (8) des Behälters (1) der Vorrichtung;
j) Optional Aufheben einer reversiblen Bindung von Partikeln (18, 19) an eine Beschichtung mindestens eines Filterelements (2, 15) der Vorrichtung, bevorzugt über einen Einfluss des Mittels (10, 11, 14) zur Veränderung des Porendurchmessers der Poren (6) des Filterelements (2);
k) Optional Wiederholen der Schritte g) bis j), bis alle Partikel (18, 19) der Flüssigkeit nach ihrer Größe separiert in getrennten Flüssigkeiten vorliegen.

13. Verfahren gemäß Anspruch 12, **dadurch gekennzeichnet, dass** das Erhöhen des Porendurchmessers der Poren (6, 16) des Filterelements (2, 15) durch mindestens einen der folgenden Schritte erfolgt:
i) Erhöhung einer Zentrifugalgeschwindigkeit einer Zentrifuge des Mittels (10, 11) zur Veränderung des Porendurchmessers der Poren (6, 16) des Filterelements (2, 15); und
ii) Verringerung einer elektrischen Spannung einer Spannungsquelle (14) des Mittels (14) zur Veränderung des Porendurchmessers der Poren (6, 16) des Filterelements (2, 15).

14. Verwendung der Vorrichtung gemäß einem der Ansprüche 1 bis 11 zur Separation von Partikeln (18, 19) unterschiedlicher Größe, die in einer Flüssigkeit vorliegen, bevorzugt zur
i) Isolation von einer oder mehreren Blutzellfraktionen aus Blut, bevorzugt zur Bereitstellung der Blutzellfraktionen für die Diagnostik und/oder zur Herstellung von Blutpräparaten, insbesondere zur Erzeugung von Zelltherapeutika; und/oder
ii) Isolation von Bakterienzellen aus Blut; und/oder
iii) Isolation von Exosomen aus Blut, Blutserum oder Biosuspensionen, bevorzugt zur Bereitstellung von Exosomen für die Diagnostik und/oder zur Herstellung von Impfstoffen; und/oder
iv) Isolation von Gewebezellen aus gemischten Gewebezellfraktionen; und/oder
v) Isolation von Zellen aus gemischten Zellsuspensionen, die von Bioreaktoren stammen, wobei die Zellen bevorzugt ausgewählt sind aus der Gruppe bestehend aus Pflanzenzellen, tierischen Zellen, menschlichen Zellen, Bakterienzellen, Hefezellen und Kombinationen hiervon.

## Claims

1. A device for separating differently sized particles (18, 19) in a liquid, comprising:
a) a receptacle (1) for receiving a liquid;
b) at least one filter element (2) including a top surface (3), a bottom surface (4), and at least one side surface (5) connecting the top surface (3) to the bottom surface (4),
wherein the filter element (2) has through-pores (6) having a defined pore diameter,
wherein the filter element (2) is arranged in the receptacle (1) so as to divide the receptacle (1), in the direction of the top surface (3) of the filter element (2), into an upper compartment (7) and, in the direction of the bottom surface (4) of the filter element (2), into a lower compartment (8), so that particles (18, 19) of a liquid in the upper compartment (7) can only reach the lower compartment (8) if they pass through the filter element (2), wherein the upper compartment (7) of the receptacle (1) has an opening (9) for receiving a liquid containing particles (18, 19),
wherein the device comprises a means (10, 11, 14) for changing the pore diameter of the pores (6) of the filter element (2),
**characterized in that** the means (10, 11, 14) for changing the pore diameter of the pores (6) of the filter element (2) is suitable for exerting a force on the filter element (2), which is either directed from the center (Z) of a surface of the filter element (2) toward the edges of this surface of the filter element (2), or in the opposite direction.

2. The device according to the preceding claim, **characterized in that** the means (10, 11, 14) for changing the pore diameter of the pores (6) of the filter element (2)
i) comprises a centrifuge; and
ii) includes at least one body (11) that is connected to an outer side of the at least one side surface (5) of the filter element (2) in a force-fit manner, optionally includes at least two bodies (11), each of which is connected to an outer side of two opposing side surfaces (5) of the filter element (2) in a force-fit manner;
the at least one body (11) being suitable for exerting, by way of a change of the rotational speed of the centrifuge, a compression force or tensile force on the at least one side surface (5) of the filter element (2), so that the pores (6) of the filter element (2) are compressed or expanded.

3. The device according to any one of the preceding claims, **characterized in that** the means (10, 11, 14) for changing the pore diameter of the pores (6) of the filter element (2)
i) comprises a centrifuge; and
ii) includes at least one body (10), preferably a plurality of bodies (10), which are arranged at the top side (3) of the filter element (2) and/or in the filter element (2), and which particularly preferably have a higher specific density and/or a higher electric charge than the particles (18, 19) to be separated, most particularly preferably the at least one body (10), preferably the plurality of bodies (10), being embodied as nanoparticles, and the nanoparticles being in particular arranged around the pores (6) of the filter element (2);
the at least one body (10) being suitable for exerting, by way of a change of the rotational speed of the centrifuge, a compression force or tensile force on the pores (6) of the filter element (2), so that the pores (6) of the filter element (2) are compressed or expanded.

4. The device according to any one of the preceding claims, **characterized in that** the means (10, 11, 14) for changing the pore diameter of the pores (6) of the filter element (2)
i) comprises an electrical voltage source (14); and
ii) comprises at least one electrically conductive layer, optionally at least two electrically conductive layers, the electrical voltage source being connected to the at least one electrically conductive layer, optionally to the at least two electrically conductive layers, in an electrically conducting manner, and the at least one electrically conductive layer being arranged at the side surface (5) of the filter element (2), preferably the two electrically conductive layers being arranged at two opposing surfaces of the at least one side surface (5) of the filter element (2) and being electrically insulated with respect to one another,
the electrical voltage source (14) being suitable for exerting a compression force or tensile force on the at least one electrically conductive layer, optionally the at least two electrically conductive layers, by way of a change of the electrical voltage, so that the pores (6) of the filter element (2) are compressed or expanded.

5. The device according to any one of the preceding claims, **characterized in that** the device comprises a control unit, which is configured to control the means (10, 11, 14) for changing the pore diameter of the pores (6) of the filter element (2), preferably in such a way that
i) a centrifugal speed of a centrifuge of the means (10, 11) for changing the pore diameter of the pores (6) of the filter element (2) is changed, preferably in such a way that the centrifugal speed is incrementally increased over the course of the separation of particles (18, 19) in a liquid, the increase in particular taking place automatically over time or manually by input of a user; and/or
ii) an electrical voltage of a voltage source (14) of the means (14) for changing the pore diameter of the pores (6) of the filter element (2) is changed, preferably in such a way that the electrical voltage is incrementally decreased over the course of the separation of particles (18, 19) in a liquid, the decrease in particular taking place automatically over time or manually by input of a user; and/or
iii) the pore diameter (6) of the pores of the filter element (2) is changed in a range of 100 nm to 100 µm; and/or
iv) the pore diameter of the pores (6) of the filter element (2) is incrementally changed, automatically over time or manually by input(s) of a user of the device, to a larger diameter, preferably from a pore diameter of 200 nm to a pore diameter of 20 µm and larger, particularly preferably from a pore diameter of 200 nm via a pore diameter of 3 µm, a pore diameter of 7 µm, a pore diameter of 8-12 µm, a pore diameter of 20 µm up to a pore diameter of 100 µm.

6. The device according to any one of the preceding claims, **characterized in that** the device includes n further filter elements (15), which are arranged on the at least one filter element (2) in the direction of the upper compartment (7) of the receptacle (1) and which in each case have through-pores (16) having a defined pore diameter, the defined pore diameter of the n filter elements (15) being larger than the defined pore diameter of the at least one filter element (2) and being larger for each of the n filter elements (15) the closer the respective filter element (15) is located in the direction of the upper compartment (8) of the receptacle (1), n preferably being an integer ≥ 2, particularly preferably an integer ≥ 3, and in particular an integer in the range of 4 to 10.

7. The device according to any one of the preceding claims, **characterized in that** the at least one filter element (2), preferably each filter element (2, 15) of the device,
i) comprises or consists of fibers that have through-pores (6, 16); and/or
ii) comprises or consists of an elastic material, preferably an elastic polymer, having through-pores (6, 16); and/or
iii) comprises or consists of an electroactive material, preferably an electroactive polymer, having through-pores (6, 16); and/or
iv) comprises or consists of a piezoelectric material, preferably a piezoelectric polymer, having through-pores (6, 16); and/or
v) comprises or consists of a material that is selected from the group consisting of silicone elastomer, thermoplastic elastomer, magnetorheological elastomer, piezoelectric elastomer, thermoplastic urethane, and combinations thereof; and/or
vi) comprises or consists of a composite material, the composite material preferably comprising or consisting of an elastomer and nanoparticles embedded therein, the nanoparticles preferably being magnetic, piezoelectric and/or gravitation-sensitive nanoparticles; and/or
vii) comprises or consists of a woven fabric or knitted fabric, the woven fabric or knitted fabric preferably comprising or consisting of a material selected from the group consisting of PES, PET, PC, PMMA, COC, nylon, glass fibers, PVDF, PP, and combinations thereof; and/or
viii) comprises or consists of a solid foam, the foam preferably comprising or consisting of a material selected from the group consisting of silicone elastomer, thermoplastic elastomer, magnetorheological elastomer, piezoelectric elastomer, thermoplastic urethane, composite material made of an elastomer having nanoparticles embedded therein, PES, PET, PC, PMMA, COC, nylon, glass fibers, PVDF, PP, and combinations thereof; and/or
ix) has an expansion from the top side (3) thereof to the bottom side (4) thereof of > 250 µm, preferably of ≥ 500 µm, particularly preferably of ≥ 1 mm, most particularly preferably of ≥ 2 mm, in particular in the range of 3 mm to 10 mm.

8. The device according to any one of the preceding claims, **characterized in that** the at least one filter element (2), preferably each filter element (2, 15), of the device, comprises a coating that is suitable for reversibly binding certain particles (18, 19) of a liquid, the coating preferably
i) comprising or consisting of a material that is suitable for being influenced by the means (10, 11, 14) for changing the pore diameter of the pores (6, 16) of the filter element (2) in such a way that the bond with the certain particles (18, 19) is dissolved, the material most particularly preferably comprising or consisting of an electroactive material, in particular an electroactive polymer; and/or
ii) being arranged at the top surface (3), bottom surface (4) and/or pore inner surface of the at least one filter element (2), particularly preferably at a top surface (3), bottom surface (4) and/or pore inner surface of all filter elements (2) of the device.

9. The device according to any one of the preceding claims, **characterized in that** the lower compartment (8) of the receptacle (1) of the device comprises a means (17) for withdrawing liquid from the lower compartment (8), preferably a valve, particularly preferably an acceleration-sensitive valve and/or a voltage-switchable valve, in particular a control unit of the device being configured to open and close the means (17) for withdrawing the liquid automatically over time, or manually by input(s) of a user.

10. The device according to any one of the preceding claims, **characterized in that** the receptacle (1) is selected from the group consisting of centrifuge tube, blood collection syringe, blood donation bag, culture bag for the biotechnological production of pharmaceuticals, bioreactor for biotechnological production, sample vessel, culture vessel, and combinations thereof.

11. The device according to any one of the preceding claims, **characterized in that** the particles (18, 19) are selected from the group consisting of vesicles, virus particles and biological cells, preferably selected from the group consisting of vesicles, virus particles and biological cells from blood, particularly preferably selected from the group consisting of en-dosomal vesicles, exosomal vesicles, virus particles, liposomes, thrombocytes, erythrocytes, leukocytes, and combinations thereof.

12. A method for separating differently sized particles (18, 19) in a liquid, comprising the following steps:
a) providing a device according to any one of claims 1 to 11;
b) adjusting the pore diameter of the pores (6, 16) of the filter element (2, 15) of the device so that either no particles (18, 19) or only particles (18) up to a desired particle diameter pass through the filter element (2, 15);
c) filling the upper compartment of the container (1) of the device with a liquid that contains particles (18, 19) having different sizes;
d) moving the liquid through the filter element (2, 15), preferably by way of a means selected from the group consisting of centrifuge, pump, and combinations thereof;
e) isolating the liquid, which optionally contains passed particles (18), from the lower compartment (8) of the receptacle (1) of the device;
f) increasing the pore diameter of the pores (6, 16) of the filter element (2) of the device so that particles (19) up to a desired pore diameter can pass through the filter element (2, 15);
g) optionally filling the upper compartment (1) of the container of the device with a liquid that preferably does not comprise any particles (18, 19);
h) moving the liquid through the filter element (2, 15), preferably by way of a means selected from the group consisting of centrifuge, pump, and combinations thereof;
i) isolating the liquid, including the passed particles (19), from the lower compartment (8) of the receptacle (1) of the device;
j) optionally dissolving a reversible bond of particles (18, 19) to a coating of at least one filter element (2, 15) of the device, preferably by an influence of the means (10, 11, 14) for changing the pore diameter of the pores (6) of the filter element (2);
k) optionally repeating steps g) to j) until all particles (18, 19) of the liquid are present in separate liquids, separated according to size.

13. The method according to claim 12, **characterized in that** the pore diameter of the pores (6, 16) of the filter element (2, 15) is increased by at least one of the following steps:
i) increasing a centrifugal speed of a centrifuge of the means (10, 11) for changing the pore diameter of the pores (6, 16) of the filter element (2, 15); and
ii) decreasing an electrical voltage of a voltage source (14) of the means (14) for changing the pore diameter of the pores (6, 16) of the filter element (2, 15).

14. Use of the device according to any one of claims 1 to 11 for separating particles (18, 19) of differing sizes present in a liquid, preferably for
i) the isolation of one or more blood cell fractions of blood, preferably for providing the blood cell fractions for diagnosis and/or for manufacturing blood preparations, in particular for producing cell therapeutics; and/ or
ii) isolating bacterial cells from blood; and/or
iii) isolating exosomes from blood, serum or biosuspensions, preferably for providing exosomes for diagnostics and/or for producing vaccines; and/or
iv) isolating tissue cells from mixed tissue cell fractions; and/or
v) isolating cells from mixed cell suspensions that originate from bioreactors, with the cells preferably being selected from the group consisting of plant cells, animal cells, human cells, bacteria cells, yeast cells, and combinations thereof.

## Revendications

1. Dispositif pour la séparation de particules (18, 19) de tailles différentes dans un liquide, comprenant
a) un récipient (1) pour recevoir un liquide ;
b) au moins un élément filtrant (2) avec une surface supérieure (3), une surface inférieure (4) et au moins une surface latérale (5) reliant la surface supérieure (3) à la surface inférieure (4), dans lequel l'élément filtrant (2) présente des pores (6) continus avec un diamètre de pore défini,
dans lequel l'élément filtrant (2) est disposé dans le récipient (1) de telle sorte qu'il divise le récipient (1) en un compartiment supérieur (7) en direction de la surface supérieure (3) de l'élément filtrant (2) et en un compartiment inférieur (8) en direction de la surface inférieure (4) de l'élément filtrant (2), de sorte que des particules (18, 19) d'un liquide dans le compartiment supérieur (7) ne puissent parvenir dans le compartiment inférieur (8) que lorsqu'elles passent l'élément filtrant (2), dans lequel le compartiment supérieur (7) du récipient (1) présente une ouverture (9) pour recevoir un liquide avec des particules (18, 19),
dans lequel le dispositif présente un moyen (10, 11, 14) de modification du diamètre de pore des pores (6) de l'élément filtrant (2),
**caractérisé en ce que** le moyen (10, 11, 14) de modification du diamètre des pores (6) de l'élément filtrant (2) est adapté pour exercer sur l'élément filtrant (2) une force qui est dirigée soit du centre (Z) d'une surface de l'élément filtrant (2) vers les bords de cette surface de l'élément filtrant (2), soit dans la direction inverse.

2. Dispositif selon la revendication précédente, **caractérisé en ce que** le moyen (10, 11, 14) de modification de la largeur de pore des pores (6) de l'élément filtrant (2)
i) présente une centrifugeuse ; et
ii) comprend au moins un corps (11) qui est relié par une liaison assurant la transmission de force à un côté extérieur de la au moins une surface latérale (5) de l'élément filtrant (2), comprend éventuellement au moins deux corps (11) qui sont reliés par une liaison assurant la transmission de force respectivement à un côté extérieur de deux surfaces latérales (5) opposées de l'élément filtrant (2) ;
dans lequel le ou au moins un corps (11) est adapté pour exercer une force de compression ou force de traction sur la ou au moins une surface latérale (5) de l'élément filtrant (2) par l'intermédiaire d'une modification de la vitesse de rotation de la centrifugeuse, de sorte que les pores (6) de l'élément filtrant (2) sont comprimés ou étirés.

3. Dispositif selon l'une quelconque des revendications précédentes, **caractérisé en ce que** le moyen (10, 11, 14) de modification de la largeur de pore des pores (6) de l'élément filtrant (2)
i) présente une centrifugeuse ; et
ii) comprend au moins un corps (10), de préférence plusieurs corps (10), qui sont disposés sur la face supérieure (3) de l'élément filtrant (2) et/ou incorporés dans l'élément filtrant (2) et qui présentent de manière particulièrement préférée une densité spécifique plus élevée et/ou une charge électrique plus élevée que les particules (18, 19) à séparer, dans lequel tout particulièrement de préférence le ou au moins un corps (10), de préférence les plusieurs corps (10), sont conçus comme des nanoparticules et les nanoparticules sont disposées en particulier autour des pores traversants (6) de l'élément filtrant (2) ;
dans lequel le au moins un corps (10) est adapté pour exercer une force de compression ou force de traction sur les pores (6) de l'élément filtrant (2) par l'intermédiaire d'une modification de la vitesse de rotation de la centrifugeuse, de sorte que les pores (6) de l'élément filtrant (2) sont comprimés ou étirés.

4. Dispositif selon l'une quelconque des revendications précédentes, **caractérisé en ce que** le moyen (10, 11, 14) de modification de la largeur de pore des pores (6) de l'élément filtrant (2)
i) présente une source de tension électrique (14) ; et
ii) présente au moins une couche électriquement conductrice, éventuellement au moins deux couches électriquement conductrices, dans lequel la source de tension électrique est reliée de manière électriquement conductrice à la au moins une couche électriquement conductrice, éventuellement aux au moins deux couches électriquement conductrices, dans lequel la ou au moins une couche électriquement conductrice est disposée sur la surface latérale (5) de l'élément filtrant (2), de préférence les deux couches électriquement conductrices sont disposées sur deux surfaces opposées de la ou au moins une surface latérale (5) de l'élément filtrant (2) et sont isolées électriquement l'une de l'autre,
dans lequel la source de tension électrique (14) est adaptée pour exercer une force de compression ou force de traction sur la ou au moins une couche électriquement conductrice, éventuellement les ou au moins deux couches électriquement conductrices, par l'intermédiaire d'une modification de sa tension électrique, de sorte que les pores (6) de l'élément filtrant (2) sont comprimés ou étirés.

5. Dispositif selon l'une quelconque des revendications précédentes, **caractérisé en ce que** le dispositif présente une unité de commande qui est configurée pour commander le moyen (10, 11, 14) de modification du diamètre de pore des pores (6) de l'élément filtrant (2), de préférence de telle sorte que
i) une vitesse de centrifugation d'une centrifugeuse du moyen (10, 11) de modification du diamètre des pores (6) de l'élément filtrant (2) est modifiée, de préférence de telle sorte que la vitesse centrifuge est augmentée progressivement au cours de la séparation de particules (18, 19) dans un liquide, dans lequel l'augmentation est effectuée en particulier automatiquement au fil du temps ou manuellement par entrée d'un utilisateur ; et/ou
ii) une tension électrique d'une source de tension (14) du moyen (14) de modification du diamètre de des pores (6) de l'élément filtrant (2) est modifiée, de préférence de telle sorte que la tension électrique est diminuée progressivement au cours de la séparation de particules (18, 19) dans un liquide, dans lequel la diminution est effectuée en particulier automatiquement au fil du temps ou manuellement par entrée d'un utilisateur ; et/ou
iii) le diamètre des pores (6) de l'élément filtrant (2) est modifié dans une plage de 100 nm à 100 µm ; et/ou
iv) le diamètre des pores (6) de l'élément filtrant (2) est modifié automatiquement au fil du temps ou manuellement par entrée(s) d'un utilisateur du dispositif par étapes jusqu'à un diamètre de pore plus grand, de préférence d'un diamètre de pore de 200 nm à un diamètre de pore de 20 µm et plus, de manière particulièrement préférée d'un diamètre de pore de 200 nm à un diamètre de pore de 100 µm en passant par un diamètre de pore de 3 µm, un diamètre de pore de 7 µm, un diamètre de pore de 8-12 µm, un diamètre de pore de 20 µm.

6. Dispositif selon l'une quelconque des revendications précédentes, **caractérisé en ce que** le dispositif comprend n autres éléments filtrants (15) qui sont disposés en direction du compartiment supérieur (7) du récipient (1) sur le au moins un élément filtrant (2) et qui présentent chacun des pores (16) continus avec un diamètre de pore défini, dans lequel le diamètre de pore défini des n éléments filtrants (15) est supérieur au diamètre de pore défini du au moins un élément filtrant (2) et est d'autant plus grand pour chacun des n éléments filtrants (15) que l'élément filtrant (15) respectif se trouve plus près en direction du compartiment supérieur (8) du récipient (1), dans lequel n est de préférence un nombre entier ≥ 2, de manière particulièrement préférée un nombre entier ≥ 3, en particulier un nombre entier dans la plage de 4 à 10.

7. Dispositif selon l'une quelconque des revendications précédentes, **caractérisé en ce que** le au moins un élément filtrant (2), de préférence respectivement tous les éléments filtrants (2, 15) du dispositif,
i) comprend ou est constitué de fibres qui présentent des pores (6, 16) continus ; et/ou
ii) comprend ou est constitué d'un matériau élastique, de préférence un polymère élastique, qui présente des pores (6, 16) continus ; et/ou
iii) comprend ou est constitué d'un matériau électroactif, de préférence un polymère électroactif, qui présente des pores (6, 16) continus ; et/ou
iv) comprend ou est constitué d'un matériau piézoélectrique, de préférence un polymère piézoélectrique, qui présente des pores (6, 16) continus ; et/ou
v) comprend ou est constitué d'un matériau qui est choisi dans le groupe constitué par les élastomère de silicone, élastomère thermoplastique, élastomère magnétorhéologique, élastomère piézoélectrique, uréthane thermoplastique et leurs combinaisons ; et/ou
vi) comprend ou est constitué d'un matériau composite, dans lequel le matériau composite comprend ou est constitué de préférence d'un élastomère et de nanoparticules incorporées dans celui-ci, dans lequel les nanoparticules sont de préférence des nanoparticules magnétiques, piézoélectriques et/ou sensibles à la gravité ; et/ou
vii) comprend ou est constitué d'un tissu de fibres ou tricot de fibres, dans lequel le tissu de fibres ou tricot de fibres comprend ou est constitué de préférence d'un matériau qui est choisi dans le groupe constitué par les PES, PET, PC, PMMA, COC, nylon, fibres de verre, PVDF, PP et leurs combinaisons ; et/ou
viii) comprend ou est constituée d'une mousse solide, dans lequel la mousse comprend ou est constituée de préférence d'un matériau qui est choisi dans le groupe constitué par les élastomère de silicone, élastomère thermoplastique, élastomère magnétorhéologique, élastomère piézoélectrique, uréthane thermoplastique, matériau composite issu d'un élastomère dans lequel sont incorporées des nanoparticules, PES, PET, PC, PMMA, COC, nylon, fibres de verre, PVDF, PP et leurs combinaisons ; et/ou
ix) présente une extension de sa face supérieure (3) à sa face inférieure (4) > 250 µm, de préférence ≥ 500 µm, de manière particulièrement préférée ≥ 1 mm, idéalement ≥ 2 mm, en particulier dans la plage de 3 mm à 10 mm.

8. Dispositif selon l'une quelconque des revendications précédentes, **caractérisé en ce que** le au moins un élément filtrant (2), de préférence respectivement tous les éléments filtrants (2, 15) du dispositif, présente un revêtement qui est adapté pour lier de manière réversible certaines particules (18, 19) d'un liquide, dans lequel le revêtement
i) comprend ou est constitué de préférence d'un matériau qui est adapté pour être influencé par le moyen (10, 11, 14) de modification du diamètre de pore des pores (6, 16) de l'élément filtrant (2) de sorte que la liaison aux particules (18, 19) déterminées soit rompue, dans lequel le matériau comprend ou est constitué idéalement d'un matériau électroactif, en particulier d'un polymère électroactif ; et/ou
ii) est disposé sur la surface supérieure (3), surface inférieure (4) et/ou surface intérieure de pores de l'au moins un élément filtrant (2), de manière particulièrement préférée sur une surface supérieure (3), surface inférieure (4) et/ou surface intérieure de pores de tous les éléments filtrants (2) du dispositif.

9. Dispositif selon l'une quelconque des revendications précédentes, **caractérisé en ce que** le compartiment inférieur (8) du récipient (1) du dispositif présente un moyen (17) de prélèvement de liquide du compartiment inférieur (8), de préférence une vanne, de manière particulièrement préférée une vanne sensible à l'accélération et/ou une vanne commandée par en tension, dans lequel en particulier une unité de commande du dispositif est configurée pour ouvrir et pour fermer le moyen (17) de prélèvement de liquide automatiquement au fil du temps, ou manuellement par entrée(s) d'un utilisateur.

10. Dispositif selon l'une quelconque des revendications précédentes, **caractérisé en ce que** le récipient (1) est choisi dans le groupe constitué par les tube de centrifugation, seringue de prélèvement sanguin, poche de don de sang, poche de culture pour la production biotechno-logique de médicaments, bioréacteur pour la production biotechnolo-gique, récipient pour échantillon, récipient de culture et leurs combinaisons.

11. Dispositif selon l'une quelconque des revendications précédentes, **caractérisé en ce que** les particules (18, 19) sont choisies dans le groupe constitué par les vésicules, particules virales et cellules biologiques ; de préférence choisies dans le groupe constitué par les vésicules, particules virales et cellules biologiques du sang, de manière particulièrement préférée choisies dans le groupe constitué par les vésicules en-dosomales, vésicules exosomales, particules virales, liposomes, thrombocytes, érythrocytes, leucocytes et leurs combinaisons.

12. Procédé de séparation de particules (18, 19) de tailles différentes dans un liquide, comprenant les étapes de
a) fourniture d'un dispositif selon l'une quelconque des revendications 1 à 11 ;
b) réglage du diamètre de pore des pores (6, 16) de l'élément filtrant (2, 15) du dispositif, de sorte que soit aucune particule (18, 19), soit seulement des particules (18) jusqu'à un diamètre de particule souhaité puissent passer l'élément filtrant (2, 15) ;
c) remplissage du compartiment supérieur du récipient (1) du dispositif avec un liquide qui comprend des particules (18, 19) avec une taille différente ;
d) déplacement du liquide à travers l'élément filtrant (2, 15), de préférence par l'intermédiaire d'un moyen choisi dans le groupe constitué par les centrifugeuse, pompe et leurs combinaisons ;
e) isolement du liquide, qui comprend éventuellement des particules (18) passées, du compartiment inférieur (8) du récipient (1) du dispositif ;
f) augmentation du diamètre des pores (6, 16) de l'élément filtrant (2) du dispositif, de sorte que des particules (19) jusqu'à un diamètre de pore souhaité puissent passer l'élément filtrant (2, 15) ;
g) éventuellement remplissage du compartiment supérieur du récipient (1) du dispositif avec un liquide qui ne comprend de préférence pas de particules (18, 19) ;
h) déplacement du liquide à travers l'élément filtrant (2, 15), de préférence par l'intermédiaire d'un moyen choisi dans le groupe constitué par les centrifugeuse, pompe et leurs combinaisons ;
i) isolement du liquide avec les particules (19) passées du compartiment inférieur (8) du récipient (1) du dispositif ;
j) éventuellement suppression d'une liaison réversible de particules (18, 19) à un revêtement d'au moins un élément filtrant (2, 15) du dispositif, de préférence par l'intermédiaire d'une influence du moyen (10, 11, 14) de modification du diamètre des pores (6) de l'élément filtrant (2) ;
k) éventuellement répétition des étapes g) à j) jusqu'à ce que toutes les particules (18, 19) du liquide soient séparés par leur taille dans des liquides distincts.

13. Procédé selon la revendication 12, **caractérisé en ce que** l'augmentation du diamètre de pore des pores (6, 16) de l'élément filtrant (2, 15) est effectuée par au moins une des étapes suivantes :
i) l'augmentation d'une vitesse de centrifugation d'une centrifugeuse du moyen (10, 11) de modification du diamètre de pore des pores (6, 16) de l'élément filtrant (2, 15) ; et
ii) la réduction de la tension électrique d'une source de tension (14) du moyen (14) de modification du diamètre de pore des pores (6, 16) de l'élément filtrant (2, 15).

14. Utilisation du dispositif selon l'une quelconque des revendications 1 à 11 pour la séparation de particules (18, 19) de différentes tailles qui sont présentes dans un liquide, de préférence pour
i) l'isolement d'une ou de plusieurs fractions de cellules sanguines du sang, de préférence pour la fourniture des fractions de cellules sanguines pour le diagnostic et/ou pour la fabrication de préparations sanguines, en particulier pour la production de médicaments de thérapie cellulaire ; et/ou
ii) l'isolement de cellules bactériennes du sang ; et/ou
iii) l'isolement d'exosomes du sang, du sérum sanguin ou de biosuspensions, de préférence pour la fourniture d'exosomes pour le diagnostic et/ou la fabrication de vaccins ; et/ou
iv) l'isolement de cellules tissulaires de fractions de cellules tissulaires mélangées ; et/ou
v) l'isolement de cellules de suspensions cellulaires mélangées qui proviennent de bioréacteurs, dans laquelle les cellules sont de préférence choisies dans le groupe constitué par les cellules végétales, cellules animales, cellules humaines, cellules bactériennes, cellules de levure et leurs combinaisons.
